# EUROPEAN PATENT APPLICATION

(11) **EP 3 311 838 A1**
(43) Date of publication of application: **25.04.2018**
(21) Application number: 16290202.7
(22) Date of filing: 21.10.2016
(51) Int. Cl.: A61K 41/00, A61K 49/18, A61P 35/00, A61K 33/26, A61K 47/69, A61K 35/74

(54) **MAGNETIC NANOPARTICLES ASSOCIATED TO IMMUNOGENIC ENTITIES FOR DESTROYING PATHOLOGICAL CELLS IN AN INDIVIDUAL**

(71) Applicant: Nanobacterie, 75116 Paris (FR)
(72) Inventor: The designation of the inventor has not yet been filed

(57) **Abstract**

This invention concerns a composition comprising at least one association of at least one magnetic nanoparticle and at least one substance, wherein the association is activated by radiation, for use in a treatment by radiation for destroying at least one pathological cell in an individual, comprising administering the composition at a body site of an individual and performing at least one type of activation of the association by radiation to activate magnetic nanoparticles without producing any temperature increase, whereby the pathological cell destruction occurs at the administration site and away from this site beyond the association diffusion region.

## Description

### FIELD OF THE INVENTION

The field of the invention is that of a composition comprising magnetic nanoparticles for destroying a pathological cell.

### TECHNICAL BACKGROUND

It has previously been shown that it was possible to destroy tumors by administering nanoparticles to tumors and by heating these nanoparticles under the application of an alternating magnetic field. Efficient tumor destruction could usually be achieved when nanoparticles are homogenously distributed within the whole tumor volume.

### DESCRIPTION OF THE INVENTION

For tumors with a non-uniform geometry such as glioblastoma tumors that possess ramifications or for metastases, it is difficult to obtain a nanoparticle distribution within the whole tumor volume. It therefore appears necessary to develop a method of tumor destruction, such as that described here in this invention, wherein magnetic nanoparticles only occupy a portion of the whole tumor volume and are activated under radiation, preferentially by being heated under the application of an alternating magnetic field. Following this activation, the whole tumor, possibly including metastases, could be destroyed by an indirect mechanism.

Such mechanism could involve the recruitment of targeting cells that specifically target pathological cells and destroy them. It may also involve the destruction of blood vessels irrigating the tumor.

As such, the present invention relates to a composition comprising at least one association of at least one magnetic nanoparticle and at least one substance, wherein the association is activated by radiation, for use in a treatment by radiation for destroying at least one pathological cell in an individual, comprising administering the composition at a body site of an individual and performing at least one type of activation of the association by radiation to activate magnetic nanoparticles without producing any temperature increase, whereby the pathological cell destruction occurs at the administration site and away from this site beyond the association diffusion region.

The present invention also relates to a method of treatment by radiation for destroying at least one pathological cell in an individual, comprising administering the individual an effective amount of a composition comprising at least one association of at least one magnetic nanoparticle and at least one substance, wherein the association is activated by radiation, wherein the composition is administered at a body site of an individual and at least one type of activation of the association by radiation is performed to activate magnetic nanoparticles without producing any temperature increase, whereby the pathological cell destruction occurs at the administration site and away from this site beyond the association diffusion region.

An individual, according to the invention, can be a living organism. An individual can also mean part of an individual, a cell, the cell, the association, the immune entity, a virus, a tumor, an organ, a vessel, an arteria, skin, or any type of biological or chemical entity that is not entirely chemically synthesized, is produced by or originates from an individual or any type of living organism.

The living organism can be a human, an animal, a plant, an archaea, a fungi, a protist, a bacterium such as a magnetotactic bacterium, a unicellular or a multicellular organism, or one or more prokaryotic or eukaryotic cell. Preferably, the living organism can be associated to part of this organism. Preferably, the living organism can synthetize the magnetic nanoparticle or the substance. In some other conditions, the living organism can synthetize the magnetic nanoparticle and the substance. Preferably also, the living organism synthesizes neither the nanoparticle nor the substance. Preferably, the living organism is the individual.

Preferably, a cell can designates a whole cell.

In some other conditions, a cell can designate part of a cell, an organelle, DNA, RNA, a protein, an enzyme, a lipid, an assembly of amino acids or nucleic acids, or any type of biological or chemical entity produced by the cell or originating from the cell.

According to the invention, the cell can be defined as the smallest unit of life, or as an assembly of biological material containing at least a nucleus, a membrane or organelles. It can be a prokaryotic or eukaryotic cell.

In an embodiment of the invention, the cell is a pathological cell. The pathological cell can be defined as a cell, which is not healthy, which is different from a cell found in a healthy individual, which is a cancerous cell, a bacterium, a cell infected by a virus, a bacterium, or a pathogen.

In another embodiment of the invention, the cell is a targeting cell. The targeting cell can be defined as a cell that specifically targets and/or destroys and/or removes the association or the cell.

In an embodiment of the invention, the targeting of the association or of the cell, preferentially by the targeting cell, can be associated with the apparition of the targeting cell at a target site, preferentially within less than 1, 10, or 60 seconds, 1, 10, or 60 minutes, 2, 10, 24, 2, 10, or 30 days, 2, or 12 months, 2, 10, or 20 years following administration of the composition.

In an embodiment, the target site can correspond to the site to which the targeting cells are to be attracted. The target site can also correspond to the site where one wants the cells to be attracted.

In an embodiment, the target site can correspond to a pathological site, an administration site or to an association diffusion region.

In an embodiment, the target set can also be designated as the site that is targeted or targeted site.

According to the invention, the pathological site can be a site comprising an assembly of more or less than 1, 2, 5, 10, 25, 50, 75, 80, or 95% of the total number of pathological cells comprised at the site of an individual. It can also be the site comprising more or less than 1, 10, 10², 10³, 10⁴, 10⁵, 10⁶, 10⁷, 10⁸, or 10⁹ pathological cell(s).

According to the invention, the administration site can be the site comprising more or less than 1, 2, 5, 10, 25, 50, 75, 80, or 95% of the total number of association comprised at the site of an individual or in the administration site. It can also be the site comprising more or less than 1, 10, 10², 10³, 10⁴, 10⁵, 10⁶, 10⁷, 10⁸, or 10⁹ association(s). The administration site can correspond to the site, where one wants to administer de composition.

According to the invention, the administration site can also correspond to the body site of an individual where the composition is administered. Such body site of an individual where the composition is administered can be one or several of the following sites: an organ, a tumor belonging to an organ, a tumor of or within an organ, an organ belonging to the musculoskeletal or muscular system, a human skeleton, a joint, a ligament, a tendon, an organ belonging to the digestive system, mouth, teeth, tongue, salivary gland, parotid gland, submandibular gland, sublingual gland, pharynx, esophagus, stomach, small intestine, duodenum, jejunum, ileum, large intestine, liver, gallbladder, pancreas, nasal cavity, pharynx, larynx, trachea, bronchi, lung, diaphragm, an organ belonging to the urinary system, kidney, ureter, bladder, urethra, an organ belonging to the male or female reproductive system, ovary, fallopian tube, uterus, vagina, vulva, clitoris, placenta, testes, epididymis, vas deferens, seminal vesicle, prostate, bulbourethral gland, penis, scrotum, an organ belonging to the endocrine glands, pituitary gland, pineal gland, thyroid gland, parathyroid glands, adrenal glands, pancreas, an organ belonging to the circulatory system, heart, arteries, veins, capillaries, an organ belonging to the lymphatic system, lymphatic vessel, lymph node, bone marrow, thymus, spleen, an organ belonging to the nervous system, the brain, cerebral hemispheres, diencephalon, the brainstem, midbrain, pons, medulla oblongata, cerebellum, the spinal cord, the ventricular system, choroid plexus, an organ belonging to the peripheral nervous system, nerves, cranial nerves, spinal nerves, a sensory organ, an eye, coronae, iris, ciliary bod, lens, retina, ear, outer ear, earlobe, eardrum, middle ear, ossicles, inner ear, cochlea, vestibule of the ear, semicircular canals, olfactory epithelium, tongue, taste buds, an organ of the integumentary system, mammary glands, laboratory-grown organ, a transplanted organ, skin, or a subcutaneous tissue.

According to the invention, the body site of an individual can correspond to, or be located at a distance of less than 10 m, 1 m, 1 cm, 1 mm, or 1 µm from: an organ, a tumor belonging to an organ, a tumor of or within an organ, an organ belonging to the musculoskeletal or muscular system, a human skeleton, a joint, a ligament, a tendon, an organ belonging to the digestive system, mouth, teeth, tongue, salivary gland, parotid gland, submandibular gland, sublingual gland, pharynx, esophagus, stomach, small intestine, duodenum, jejunum, ileum, large intestine, liver, gallbladder, pancreas, nasal cavity, pharynx, larynx, trachea, bronchi, lung, diaphragm, an organ belonging to the urinary system, kidney, ureter, bladder, urethra, an organ belonging to the male or female reproductive system, ovary, fallopian tube, uterus, vagina, vulva, clitoris, placenta, testes, epididymis, vas deferens, seminal vesicle, prostate, bulbourethral gland, penis, scrotum, an organ belonging to the endocrine glands, pituitary gland, pineal gland, thyroid gland, parathyroid glands, adrenal glands, pancreas, an organ belonging to the circulatory system, heart, arteries, veins, capillaries, an organ belonging to the lymphatic system, lymphatic vessel, lymph node, bone marrow, thymus, spleen, an organ belonging to the nervous system, the brain, cerebral hemispheres, diencephalon, the brainstem, midbrain, pons, medulla oblongata, cerebellum, the spinal cord, the ventricular system, choroid plexus, an organ belonging to the peripheral nervous system, nerves, cranial nerves, spinal nerves, a sensory organ, an eye, coronae, iris, ciliary bod, lens, retina, ear, outer ear, earlobe, eardrum, middle ear, ossicles, inner ear, cochlea, vestibule of the ear, semicircular canals, olfactory epithelium, tongue, taste buds, an organ of the integumentary system, mammary glands, laboratory-grown organ, a transplanted organ, skin, or a subcutaneous tissue.

According to the invention, the administration site can also correspond to the pathological site.

According to the invention, a target site can be the site comprising an assembly of more or less than 1, 2, 5, 10, 25, 50, 75, 80, or 95% of the total number of targeting cells comprised at the body site of an individual. It can also be the site comprising more or less than 1, 10, 10², 10³, 10⁴, 10⁵, 10⁶, 10⁷, 10⁸, or 10⁹ targeting cell(s).

In one embodiment of the invention, the target site corresponds to the site that one wants to target with the targeting cell. The target site can correspond to the pathological site.

According to the invention, the body site of an individual can be a site comprising more or less than 1, 2, 5, 10, 10², 10³, 10⁴, 10⁵, 10⁶, 10⁷, 10⁸, or 10⁹ cell(s), association(s), immune entity(ies) tissue(s), organ(s), organelle(s), biological substance(s), protein(s), enzyme(s), lipid(s), DNA, or RNA, belonging to an individual or originating from an individual. The body site of an individual can comprise more than 1, 10, 10², 10³, 10⁴, 10⁵, 10⁶, 10⁷, 10⁸, or 10⁹ non healthy cells, cancerous cells, bacteria, or viruses. The body site of an individual can represent the whole individual or part of an individual. Such body site of an individual can be or represent one or several of the following sites: an organ, a tumor belonging to an organ, a tumor of or within an organ, an organ belonging to the musculoskeletal or muscular system, a human skeleton, a joint, a ligament, a tendon, an organ belonging to the digestive system, mouth, teeth, tongue, salivary gland, parotid gland, submandibular gland, sublingual gland, pharynx, esophagus, stomach, small intestine, duodenum, jejunum, ileum, large intestine, liver, gallbladder, pancreas, nasal cavity, pharynx, larynx, trachea, bronchi, lung, diaphragm, an organ belonging to the urinary system, kidney, ureter, bladder, urethra, an organ belonging to the male or female reproductive system, ovary, fallopian tube, uterus, vagina, vulva, clitoris, placenta, testes, epididymis, vas deferens, seminal vesicle, prostate, bulbourethral gland, penis, scrotum, an organ belonging to the endocrine glands, pituitary gland, pineal gland, thyroid gland, parathyroid glands, adrenal glands, pancreas, an organ belonging to the circulatory system, heart, arteries, veins, capillaries, an organ belonging to the lymphatic system, lymphatic vessel, lymph node, bone marrow, thymus, spleen, an organ belonging to the nervous system, the brain, cerebral hemispheres, diencephalon, the brainstem, midbrain, pons, medulla oblongata, cerebellum, the spinal cord, the ventricular system, choroid plexus, an organ belonging to the peripheral nervous system, nerves, cranial nerves, spinal nerves, a sensory organ, an eye, coronae, iris, ciliary bod, lens, retina, ear, outer ear, earlobe, eardrum, middle ear, ossicles, inner ear, cochlea, vestibule of the ear, semicircular canals, olfactory epithelium, tongue, taste buds, an organ of the integumentary system, mammary glands, laboratory-grown organ, a transplanted organ, skin, or a subcutaneous tissue.

In one embodiment of the invention, the body site of an individual corresponds to the administration site.

In one embodiment of the invention, an origin site is the site from which the targeting cells originate, before they are attracted to or migrate towards the target site.

In an embodiment of the invention, the removal of the association or of the cell, preferentially by the targeting cell, can be associated with the disappearance of the association from the administration site, preferentially within less than 1, 10, or 60 seconds, 1, 10, or 60 minutes, 2, 10, 24, 2, 10, or 30 days, 2, or 12 months, 2, 10, or 20 years following administration of the composition.

In an embodiment of the invention, the destruction of the pathological cell, preferentially by the targeting cell, can be partial or total and can be associated with a loss of activity, a decrease in proliferation, an inhibition, an apoptotic death, or a necrotic death, of the pathological cell.

It can also be associated with the destruction of the membrane or of any type of organelle, DNA, RNA, protein, amino acid, nucleic acid, lipid, or biological material, produced by or originating from the pathological cell. It can preferentially occur within less than 1, 10, or 60 seconds, 1, 10, or 60 minutes, 2, 10, 24, 2, 10, or 30 days, 2, or 12 months, 2, 10, or 20 years following administration of the composition.

In still another embodiment of the invention, the association or the cell can comprise an assembly of more or less than 1, 2, 5, 10, 10², 10³, 10⁶, 10¹⁰, 10²⁰, or 10⁴⁰ association(s) or cell(s) respectively.

In an embodiment of the invention, the environment surrounding the association is defined as the volume comprising the association and additional chemical or biological entities that do not belong to the association and are separated from the association by a distance of less than 10 m, or 1 m, or 1 cm, or 100 mm, 10 mm, 1 mm, or 100 µm, 10 µm, 1 µm, or 100 nm, or 10 nm, from the association.

In an embodiment of the invention, the environment surrounding the cell is defined as the volume comprising the cell and additional chemical or biological entities that do not belong to the cell and are separated from the cell by a distance of less than 10 m, or 1 m, or 1 cm, or 100 mm, 10 mm, 1 mm, or 100 µm, 10 µm, 1 µm, or 100 nm, or 10 nm from the cell.

In one embodiment of the invention, the composition is administered following at least one of the following different routes: gastrointestinal, enteral, through the gastrointestinal tract, through the intestine, through or within one of the organs of an individual, orally, into the rectum, in the stomach, sublingual, buccal, enteral, systemically, topical, epidural, intracerebral, intra-cerebroventricular, transderma, extra-amniotic, nasal, intra-arterial, intraarticular, intracardiac, intravenous, intracavernous, intradermal, intralesional, intramuscular, intraocular, into the bone marrow, intraperitoneal, intrathecal, intrauterine, intravitreal, subcutaneous, transdermal, transmucosal, intratumoral, directly in the tumor, or peritumoral, near the tumor.

Preferably, in an embodiment of the invention, the activation by radiation corresponds to the activation by radiation of the association, of the magnetic nanoparticle, of the substance, of the cell, of the pathological cell, of the targeting cell, or of an immune entity.

In another embodiment of the invention, the activation by radiation corresponds to a radiation or the application of a radiation, preferentially within the administration site, or within the target site, or within the pathological site, or within the body site of an individual, or within the origin site.

In an embodiment of the invention, the composition consists of the association.

In another embodiment of the invention, the association comprises or consists of the magnetic nanoparticle. Preferably, the association can comprise or consist of the magnetic nanoparticle without comprising the substance.

In another embodiment of the invention, the association comprises or consists of the substance.

Preferably, the association can comprise or consist of the substance without comprising the magnetic nanoparticle.

Preferably, in an embodiment of the invention, the activation by radiation corresponds to the activation by radiation of the association, of the magnetic nanoparticle, of the substance, of the cell, of the pathological cell, of the targeting cell, or of an immune entity.

In some another embodiment of the invention, the activation by radiation corresponds to a radiation or the application of a radiation, preferentially within the administration site, or within the cell site, or within the target site, or within the pathological site, or within the body site of an individual.

In still another embodiment of the invention, the activation by radiation corresponds to: (i), the migration of the targeting cells towards the target site, (ii), the destruction of the pathological or targeting cell, preferentially the destruction of the pathological cell without the destruction of the targeting cell, wherein the destruction is preferentially caused by the cell, most preferentially by the targeting cell, (iii), the removal of the pathological cell or association by the targeting cell from the targeting or administration site, or (iv), the detection of the targeting cell.

In still another embodiment of the invention, the activation by radiation corresponds to a physico-chemical perturbation, preferentially a physico-chemical perturbation of the association, of the magnetic nanoparticle, of the substance, of the cell, of the pathological cell, of the targeting cell, of the immune entity.

In another embodiment of the invention, the immune entity is the magnetic nanoparticle, the substance, the cell, an individual, or a living organism.

In still another embodiment of the invention, the immune entity is an entity belonging to the primary (innate) or secondary (adaptive) immune system.

In still another embodiment of the invention, the immune entity is one or more amino acids, an acid such as uric acid, an antigen, an antibody, a base such as NaOH, a cluster such as a cluster of differentiation, CpG, a complex such as a major histocompatibility complex, MHC, MHC-1, MHC-2, MHC-3, a cytokine, a cytoplasmic molecule such as HMGB1, DNA, preferentially bacterial DNA, an endotoxin, an enzyme, flagellin, glycan, glycoconjugate, a ligand such as a ligand expressed at the surface of stressed cells, an interleukin, a lipid, a lipopolysaccharide, a lipoteichoic acid, a protein, a stress protein, a heat shock protein, a formylated protein, RNA, a pathogen-associated molecular pattern (PAMP), peptidoglucan, a receptor, such as a molecular pattern recognition receptor (PRR), a specific Toll-like receptor (TLR), a NOD-like receptor (NLR), a RIG-I-like receptor (RGR), or a C-type lectin receptor (CLR).

In still another embodiment of the invention, the immune entity is an immune cell belonging to the immune system, an antigen presenting cell (APC), a basophil, a dendritic cell, an eosinophil, a granulocyte, a killing cell, a leukocyte, a lymphocyte, a macrophage, a mast cell, a natural killer, a neutrophil, a phagocyte, a B or T cell. An immune entity can be a cell belonging to the primary immune system, such as a phagocyte including monocyte, a macrophage, a dendritic cell, a granulocyte, a killer cell, a mast cell, or a resident cell. An immune entity can also be a cell belonging to the secondary immune system such as a leukocyte, a lymphocyte, a T or B lymphocyte, a CD8+ T lymphocyte, a helper cell (Th1 or Th2), or a gamma delta T cell.

In still another embodiment of the invention, the immune entity can be preferably and not be in some other conditions an entity, which is responsible for the progressive loss of antitumor activity. Such immune entity could be a Treg cell, which can be responsible for the progressive loss of antitumor activity of CD8+ T cells, or a cell, such as Th2, that can induce macrophage polarization towards M2 phenotype that can produces proangiogenic and immunosuppressive cytokines.

In still another embodiment of the invention, the immune entity is preferentially an immune entity involved in the destruction of the pathological cell, such as CD4+, T cell, CD8+, or a natural killer cell.

In an embodiment of the invention, the immune entity can be a naive cell, preferentially a cell that has not yet met its antigen and is inactivated.

In another conditions, an immune entity can be an effector cell, preferentially a cell that has met its antigen and is activated.

In another embodiment of the invention, the immune entity is synthesized by a living organism or comprised within a living organism, wherein an immune entity synthesized by a living organism involves a least one fabrication step by a living organism.

In still another embodiment of the invention, the immune entity is synthesized chemically, which means that none of its fabrication steps involves a living organism.

According to the invention, it is preferred that the immune entity is produced by or comes from an organ such as the lymphoid organ, thymus, bone marrow, spleen, tonsils, lymph vessel, lymph node, adenoids, skin, or liver.

In an embodiment of the invention, the immune entity is the targeting or pathological cell. According to this invention, the immune entity can designate an assembly of more than 1, 10, 10², 10⁵, 10²⁰, or 10⁴⁰ immune entities, which have similar functions or similar compositions.

According to the present invention, another immune entity can designate an assembly of more than 1, 10, 10², 10⁵, 10²⁰, or 10⁴⁰ immune entities that have different functions or different compositions from each other or from the immune entity.

According to the present invention, the immune entity can be a message that activates another immune entity.

In an embodiment of the invention, the activation, preferentially the activation by radiation, can be associated with the transition from an inactivated to an activated state of the association, of the cell, or of the immune entity. In the inactivated state, the association, the cell, or the immune entity, can remain without any physico-chemical perturbation, without any movement, without destroying, attracting, or detecting any cell or any immune entity, without being destroyed, being attracted or being detected by any cell or by any immune entity. By contrast, in the activated state, the association, the cell, or the immune entity, can be exposed to a physico-chemical perturbation, can move, destroy, or attract the cell or the immune entity, or can be moved, destroyed, or attracted by the association, the cell, or the immune entity.

In an embodiment of the invention, the transition from the inactivated to the activated state can take more or less than 1 µs, 1 millisecond, 1 second, 1 hour, 1 day, 1 month, or 1 year.

In another embodiment of the invention, the activation by radiation can be selective and induce a transition from the inactivated to the activated state of one to three of the four following elements, preferentially without inducing a transition among the other elements: (i), the magnetic nanoparticle, (ii), the substance, (iii), the cell, (iv), the immune entity. For example, the application of an alternating magnetic field could preferentially heat the magnetic nanoparticle without heating the substance, the cell, or the immune entity.

In another embodiment of the invention, the activation by radiation can be associated with the following four sequences of activation such as: (i), first, the activation of the magnetic nanoparticle, for example leading to nanoparticle heating, preferentially occurring within or outside of the association or administration site, (ii), second, the activation of the substance, for example resulting in the dissociation of the substance from the magnetic nanoparticle and/or in the chemical modification of the substance, preferentially occurring within or outside of the association or administration site, (iii), third, the activation of the targeting cell, for example yielding the destruction of the pathological cell or yielding the removal of the pathological cell from the pathological site, preferentially occurring within or outside of the target site, (iv), fourth, the activation of the pathological cell, for example resulting in the movement of the pathological cell, preferentially the removal of the pathological cell from the pathological site, or leading to the self-destruction of the pathological cell, preferentially occurring within or outside of the pathological site.

In an embodiment of the invention, the activation by radiation leads to the destruction of more than 1, 10, 10², 10³, 10⁴, 10⁵, 10⁶, 10⁷, 10⁸, or 10⁹ pathological or targeting cells.

According to the invention, at least two of these four sequences of activation can follow each other. The dissociation or modification of the substance can be due to the activation of the magnetic nanoparticle. The activation of the targeting cell can be due to that of the substance, for example when endotoxins are dissociated from magnetic nanoparticle, following the application of an alternating magnetic field and leading to the migration of polynuclear neutrophils towards endotoxins. The destruction of the pathological cell can be caused by the migration of the targeting cell towards the pathological cell such as that of polynuclear neutrophils or of other types of immune entity towards the tumor cell.

In another embodiment of the invention, the destruction of the cell, preferentially of the pathological cell, or of the association, represents a modification of the cell or of the association, for example a loss of at least 1, 10, 10², 10³, 10⁴, 10¹⁰, or 10²⁰ atom(s), protein(s), enzyme(s), DNA, RNA, or of any type of chemical or biological entity produced by the association or by the cell or originating from the association or from the cell.

According to the invention, the composition or the association can be in a liquid, solid, or gaseous state. The composition or the association may be used for a medical, clinical, preclinical, veterinary, diagnosis, cosmetic, or research purpose, for any type of treatment or diagnosis on the individual or on any type of living organism.

According to the invention, the composition or the association can be a drug, a medical device, a biological product, a cosmetic product, a therapeutic product, or a diagnostic product. Preferably, a product is defined as the composition ready for sale or abiding by regulation.

In an embodiment of the invention, the composition either comprises the association alone or the association and a vehicle. Such vehicle can favor the administration, contact, or interaction, of the association to or with an individual, part of this individual, a cell, or any type of biological or chemical entity. Preferably, it can reduce the toxicity or improve the efficacy of the association. It can favor the contact, interaction, mixing, or administration, of the association with or to an individual. Preferably, it can be an excipient, a cream, a liquid, a gas, or a solid matrix.

According to the invention, the association can be made of magnetic nanoparticles and/or substances before or after any single or multiple fabrication step(s) of the association, before or after any type of use of the association, before or after administration of the association to an individual.

Preferably, in one embodiment of the inventions, the substance can be linked to the magnetic nanoparticle by weak bonds, which can be hydrogen bonds or van der Waals interactions.

Preferably, the association is such that the substance can be linked to the magnetic nanoparticle by strong bonds, which can be ionic or covalent bonds. Strong bonds may be involved when the substance coats the magnetic nanoparticle while weak bonds may be involved when the substance is adsorbed at the surface of the magnetic nanoparticle or is located in the solid, liquid, or gaseous environment surrounding the magnetic nanoparticle. It may be possible that before its use or before its activation by radiation or before being administered to an individual, the substance is linked to the magnetic nanoparticle by a different type of bond than during or after its use, its activation by radiation or its administration to an individual.

In an embodiment of the invention, the magnetic nanoparticle is a diamagnetic, superparamagnetic, ferromagnetic or ferromagnetic nanoparticle, at physiological temperature or when it is at the body site of an individual.

In still another embodiment of the invention, the magnetic nanoparticle is the immune entity.

According to the invention, the magnetic nanoparticle can be characterized by at least one of the following properties: i), a coercivity, Hc, which is larger than 10⁻⁴, 10⁻³, 10⁻², 10⁻¹, 1, 10, 10², 10³, 10⁴, or 10⁵ Oe, or by, ii), a ratio between remanent and saturating magnetization, Mr/Ms, which is larger than 0.001, 0.01, 0.1, 0.2, 0.3, 0.4, 0.5, 0.6, 0.7, 0.8, or 0.9, or by, iii), a saturating magnetization, Ms, which is larger than 0.1, 1, 10, or 100 emu/g. Hc and/or Mr/Ms and/or Ms is/are preferentially measured at a higher temperature than 0, 10, 100, 200, 300, 400, 500, 700, or 1000 K.

In an embodiment of the invention, the magnetic nanoparticle comprises at least 1, 10, 10², 10³, 10⁴, 10⁵, 10⁶, 10⁷, 10⁸, or 10⁹ magnetic or metallic atoms. Such atom(s) can be iron, zinc, manganese, cobalt, nickel. Such atom(s) preferentially possess(es) a low toxicity or is/are combined with other atom(s) to yield a low toxicity. The magnetic nanoparticle can be made of a metal oxide, such as an iron oxide, in particular maghemite or magnetite.

According to the invention, the substance can coat the magnetic nanoparticle. In this case, the substance can have a thickness of more or less than 0.5, 1, 2, 3, 4, 5, 6, 7, 8, 9, 10, 15, 20, 25, 30, 40, 50, 75, 100, 250, 500, or 1 µm. The substance can prevent the aggregation of the magnetic nanoparticle, enable its uniform heating or yield an organization of magnetic nanoparticle in a different geometry than a cluster or aggregate, preferentially yield an anisotropic structure such as a chain.

According to the invention, the substance can also be contained in the liquid, solid or gaseous environment surrounding the magnetic nanoparticle in the composition.

In an embodiment of the invention, the substance is the immune entity.

In an embodiment of the invention, a treatment by radiation results from or is associated with the activation by radiation.

In another embodiment of the invention, the treatment by radiation is a treatment of a disease, which involves the use of radiation or the activation by radiation to cure this disease, in part or fully, and could result in complete remission, in an increase of survival, in less suffering or damages, or in improved living conditions of an individual.

In yet another embodiment of the invention, the treatment by radiation corresponds to the treatment or to any type of treatment.

In an embodiment of the invention, a treatment by radiation is a treatment of an infectious disease, a disease which involves cells or any type of biological or chemical entity that do not belong to a living healthy individual, for example cancer cells, viruses or pathogenic bacteria, and develop in this individual to weaken, or destroy, partly or fully, this individual.

In an embodiment of the invention, a treatment by radiation is a treatment of a cancer such as: adrenal, anal, bile duct, bladder, bone, brain, breast, cervical, colon/rectum, endometrial, esophagus, eye, gallbladder, kidney, laryngeal and hypopharyngeal, leukemia, liver, lung, nasal cavity and paranasal sinus, nasopharyngeal, neuroblastoma, non-hodgkin lymphoma, oral cavity and oropharyngeal, osteosarcoma, ovarian, pancreatic penile, prostate, retinoblastoma, rhabdomyosarcoma, salivary gland, sarcoma, skin Cancer, small intestine, stomach, testicular, thymus, thyroid, uterine Sarcoma, vaginal, or vulvar cancer, or a tumor such as: waldenstrom macroglobulinemia wilms tumor, castleman disease ewing family of tumor, gastrointestinal carcinoid tumor, gastrointestinal stromal tumor, myelodysplastic syndrome pituitary tumor, or a cancerous disease such as gestational trophoblastic disease, hodgkin disease, kaposi sarcoma, malignant mesothelioma, or multiple myeloma. A cancer, tumor or cancerous disease can belong to an adult, adolescent, child, animal or any other type of living organism. It can be at different grade, be localized or metastatic. It can be developed or pre-exist, be genetically programmed.

In an embodiment of the invention, one type of activation by radiation does not produce any temperature increase. This could either mean that the activation by radiation produces a temperature increase but at such a small scale that it can't be measured with so-called standard thermometry methods, or that it does not produce any thermal perturbation.

In an embodiment of the invention, one type of activation by radiation that does not produce any temperature increase can produce a temperature increase of less than 10, 5, 2, 1, 0.5, 0.1, 0.01, 0.001, or 0.0001 °C. It can also produce a temperature increase that can't be measured due to the difficulty to position the temperature probe at the administration site.

In an embodiment of the invention, one type of activation by radiation that does not produce any temperature increase does not produce a temperature that activates the targeting cell or destroys the pathological cell.

In another embodiment of the invention, one type of activation by radiation could produce a physico-chemical perturbation different from a thermal perturbation.

In still another embodiment of the invention, a thermal perturbation is a physico-chemical perturbation that produces a temperature increase of more or less than 0.01, 0.1, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 1000, or 10000 °C.

In still another embodiment of the invention, the physico-chemical perturbation different from the thermal perturbation is: (i), the movement of the association by more than 1 nm, 10 nm, 100 nm, 1 µm, 10 µm, 100 µm, 1 mm, 10 mm, 1 cm, 10 cm or 1 m, (ii), the release of the substance from the magnetic nanoparticle, (iii), a change in the composition of the association, (iv), a variation in the pH of the association by more or less than 14, 13, 12, 11, 10, 9, 8, 7, 6, 5, 4, 3, 2, or 1 pH unit(s), (v), a variation in redox potential of the association by more or less than 1 V, 100, 10, 1, or 0.1 mV, or (vi), a variation in the environment surrounding the association or the cell, such as a variation in pH, temperature, redox potential, or chemical composition of this environment.

In still another embodiment of the invention, the physico-chemical perturbation is the thermal perturbation or the physico-chemical perturbation different from the thermal perturbation.

According to the invention, the physico-chemical perturbation can occur at the administration site, or at the pathological site, or at the target site, or at the body site of an individual, or within the environment of the cell, or within the environment of the association.

In an embodiment of the invention, the physico-chemical perturbation can be due to the exposition of the association, pathological or targeting cell, to the radiation.

In an embodiment of the invention, the exposition of the association, pathological or targeting cell, to the radiation corresponds to the activation by radiation.

In another embodiment of the invention, one type of activation by radiation does not produce any temperature increase or any thermal perturbation. This could mean that the activation by radiation produces a temperature increase of less than 0.01, 0.1, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, or 100 °C above the temperature reached before the application of the radiation.

In an embodiment of the invention, the temperature before the activation by radiation can be the physiological temperature, the temperature of the individual, the temperature before the activation by radiation at the administration site, a temperature equal to, larger or lower than - 300, -200, -100, 0, 10, 20, 25, 30, 35, 37, 38, 39, 40, 41, 42, 43, 50, 60, 80, 90, 100, 200, 500, or 1000 °C.

According to the invention, a standard thermometry method can be a method that measures the temperature at a scale which is typically larger than the cellular or micrometric scale. Such method could involve thermocouples, thermistors, resistance temperature detector, or infrared camera.

In an embodiment of the invention, the composition is administered at the administration site.

According to the invention, the administration site can correspond to more or less than 1, 2, 5, 10, 25, 50, 75, 80, or 95% of the body site of an individual.

In another embodiment of the invention, it happens that following administration of the composition to an individual, part of the association leaves the body site of an individual. In this case, the administration site can preferentially comprise less than 1, 2, 5, 10, 25, 50, 75, 80, or 95% of the administered association, 1, 5, 10, or 60 seconds, 2, 5, 15, 30, or 60 minutes, 2, 5, 10, or 24 hours, 2, 5, 15, or 30 days, 2, 6, or 12 months, 2, 5, or 10 years following administration of the association.

Preferably, the administered suspension corresponds to the suspension administered to an individual and/or to the suspension contained within a tube, matrix, container, volume, or space and/or to the suspension before, during, or after its administration to an individual.

In another embodiment of the invention, to enable the treatment, the administration site comprises a sufficiently large percentage of administered association, preferentially more than 1, 2, 5, 10, 25, 50, 75, 80, or 95% of the administered suspension. To enable the treatment, the association can also remain within the administration site for a sufficiently long time, preferentially a longer time than 1, 5, 10, or 60 seconds, 2, 5, 15, 30, or 60 minutes, 2, 5, 10, or 24 hours, 2, 5, 15, or 30 days, 2, 6, or 12 months, 2, 5, or 10 years following administration of the association.

In another embodiment of the invention, the protocol of fabrication and administration of the composition to an individual is optimized to lead during a sufficiently long time to a sufficiently high percentage of the administered composition at the administration site or at the body site of an individual to enable the treatment. At the same time, this protocol is also optimized such that the administered composition leaves the administration site or the body site of an individual, sufficiently rapidly to avoid potential toxicity.

In an embodiment of the invention, the composition is administered at a pathological site or is comprised within the pathological site.

In an embodiment of the invention, the composition is administered at a healthy site or is comprised within the healthy site. The healthy site can be a site comprising an assembly of more than 1, 10, 10², 10³, 10⁴, 10⁵, 10⁶, 10⁷, 10⁸, or 10⁹ healthy cell(s) or a site comprising less than 1, 10, 10², 10³, 10⁴, 10⁵, 10⁶, 10⁷, 10⁸, or 10⁹ pathological cell(s).

In an embodiment of the invention, the composition is administered at a target site or is comprised within the target site.

According to the invention, the body site of an individual, the target site, the healthy site, or the pathological site, can correspond to the environment of the association or to the environment of the cell.

In an embodiment of the invention, the composition is administered at the body site of an individual or comprised within the body site of an individual.

In an embodiment of the invention, the association diffusion region comprises the body site of an individual and/or the administration site. The association diffusion region can comprise 1,01, 1,1, 1,2, 1,5, 2, 5, 10, 50, 10², 10³, 10⁴, 10⁵, 10¹⁰, or 10²⁰ times more associations than the administration site or can encompass a volume 1,01, 1,1, 1,2, 1,5, 2, 5, 10, 50, 10², 10³, 10⁴, 10⁵, 10¹⁰, or 10²⁰ times larger than that of the administration site. Such site can also correspond to the volume or space surrounding the association and other biological or chemical entities that do not belong to the association, located at a distance from the administration site of less than 1 m, 100 cm, 10 cm, 1 cm, 1 mm, 100 µm, 10 µm, 1 µm, 100 nm, or 10 nm.

In an embodiment of the invention, the targeting or the pathological cell can be considered as away from the administration site when it is located at more than 1, 10, or 100 nm, 1, 10, or 100 µm, or 1 mm, 1, 10, or 100 cm from the administration site.

In an embodiment of the invention, the destruction of the targeting or pathological cell occurs at the administration site and/or at a distance of more or less than 10 nm, or 100 nm, 1 µm, 10 µm, or 100 µm, or 1 mm, 1 cm, 10 cm, or 100 cm, or 1 m from this site. This is for example the case when the destruction of the pathological cell involves the destruction of metastases, which are far from the primary tumor site. Preferably, metastases are pathological cells which are disseminated within the body site of an individual at a cellular concentration, which is preferentially below 1, 2, 5, 10, 10², 10³, 10⁴, or 10⁵ pathological cell(s) per cm³. In some other conditions, the primary tumor site is the body site of an individual comprising more than 1, 2, 5, 10, 10², 10³, 10⁴, 10⁵ pathological cell(s) per cm³.

In an embodiment of the invention, the composition can be administered to an individual by a route that leads to direct contact between the association and the pathological or targeting cell such as an intratumor administration route or by a route in which the association is transported though the blood towards the pathological or targeting cell.

In an embodiment of the invention, the association, following its administration, occupies a site comprising less than 90, 80, 70, 60, 50, 40, 30, 20, 10, 5, 2, 1, 0.1, 10⁻¹, 10⁻², 10⁻³, 10⁻⁴, 10⁻⁵, 10⁻⁶, 10⁻⁷, 10⁻⁸, or 10⁻⁹ % of the total number of pathological or targeting cells, where this percentage can correspond to the number of pathological or targeting cells comprised in the association diffusion region divided by the total number of pathological or targeting cells comprised at the body site of an individual.

In an embodiment of the composition for use according to the invention, another type of activation by radiation is applied to produce a temperature increase. Such temperature increase can be a temperature increase of more than 0.01, 0.1, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, or 100°C above the temperature reached before the activation by radiation or above the physiological temperature or above the temperature of the pathological or target site or above the temperature of the body site of an individual, which is reached before the activation by radiation.

In an embodiment of the invention, another type of activation by radiation that is applied to produce a temperature increase induces a temperature increase that activates the targeting cell and/or destroys the pathological cell.

In an embodiment of the composition for use, according to the invention, the association is immunogenic.

In another embodiment of the invention, the association is immunogenic when it is the immune entity.

In another embodiment of the invention, the association is immunogenic when the magnetic nanoparticle or the substance is the immune entity.

According to the invention, the association is immunogenic when the immune entity is activated.

Preferably, the immune entity is activated when it is exposed to a radiation or when it is activated by radiation.

Preferably, the immune entity is activated when it modifies the shape, the geometry, the size, or the cellular interactions, of the association or of the cell.

Preferably, the immune entity is activated when it activates an innate or adaptive immune response, preferentially an immune response that destroys or favors the destruction of the pathological cell.

Preferably also, the immune entity is activated when it targets and/or removes and/or destroys the pathological cell. The immune entity can either target and/or destroy the pathological cell directly when it is the only activated immune entity or it can target and/or destroy the pathological cell indirectly when it triggers the activation of more than 1, 10, 10², 10⁵, 10²⁰, or 10⁴⁰ other immune entities that target and/or destroy the pathological cell.

Preferably still, the immune entity is activated when it produces other immune entities or when it increases the number of immune entities, preferentially at the pathological site, target site, body site of an individual, or association diffusion region.

In still other conditions, the immune entity is activated when it is released by a cell, preferentially by the pathological or targeting cell, preferentially by the cell under stress. Such immune entity can be a cytoplasmic molecule, such as HMGB1, uric acid or a heat shock protein such as the HSP-70.

Preferably, the immune entity is activated when it inhibits angiogenesis, induces cytotoxicity, preferentially cytotoxicity towards the pathological cell, or phagocytosis, preferentially phagocytosis of the pathological cell.

Preferably also, the immune entity is activated when it stimulates the expression of specific protein. In this case, the immune entity can be a ligand, such as a tumor necrosis factor-related apoptosis-inducing ligand (TRAIL), which could stimulate the expression of specific proteins, such as membrane protein, which may trigger the destruction of the pathological cell.

Preferably still, the immune entity is activated when it activates interleukins or cytokines, for example interferon gamma (IFN-gamma) or IL-6, or when it activates Th1 cell, lymphocyte, neutrophil, polynuclear neutrophil (PNN).

Preferably, the immune entity is activated when it is recognized by a specific receptor or when it is bound to a specific receptor, such as a toll like receptor (TLR), preferentially a TLR-9, a formyl peptide receptor (HRPF), a pattern recognition receptor, a protein, a lipid, an enzyme or any type of biological or chemical complex that can bind, preferentially specifically, to the immune entity.

In an embodiment of the invention, the association or immune entity is immunogenic when it is exposed to a radiation or activated by radiation and is non-immunogenic when it is not exposed to a radiation or when it not activated by radiation.

In an embodiment of the invention, the magnetic nanoparticle or the immune entity can be immunogenic when it is exposed to a radiation or activated by radiation, preferentially when it induces a perturbation in the environment surrounding the association or immune entity such as a the physico-chemical perturbation in this environment.

In another embodiment of the invention, the substance or the immune entity can be non-immunogenic when it is not activated by radiation and can become immunogenic when it is activated by radiation, preferentially when it dissociates from the magnetic nanoparticle and/or when it is prone to a chemical modification under radiation.

An immune entity that is non-immunogenic can be an immune entity that either activates the immune system without participating in the destruction of the pathological cell or an immune entity that does not activate the immune system and does not destroy the pathological cell.

In an embodiment of the composition for use according to the invention, the activation by radiation leads to the migration of the targeting cell towards the pathological site, wherein the targeting cell is a cell that specifically targets the pathological site.

In another embodiment of the composition for use according to the invention, the activation by radiation leads to the migration of the targeting cell towards the association, wherein the targeting cell is a cell that specifically targets the association.

In an embodiment of the invention, the targeting cell is the immune entity.

According to the invention, the targeting cell migrates towards the pathological or administration site after activation by radiation, where the activation by radiation leads to the physico-chemical perturbation that triggers the migration of the targeting cell towards the pathological or administration site.

In an embodiment of the invention, the migration of the targeting cell towards the pathological or administration site is considered the same as the attraction of the targeting cell by the pathological cell or association.

In another embodiment of the invention, the migration of the targeting cell towards the pathological cell or association is considered different from the attraction of the targeting cell by the pathological cell or association. It can occur before or after the attraction of the targeting cell by the pathological cell or association.

Preferably, the targeting cell specifically targets the administration or pathological site when it specifically targets and/or destroys an immune entity, such as an antigen, belonging to or originating from the pathological cell.

In some other conditions, the targeting cell specifically targets the administration or pathological site when it comprises or produces an immune entity, such as an antibody, that specifically targets and/or destroys the pathological cell.

In another embodiment of the invention, the activation by radiation can lead to the transport of the immune entity from the administration or pathological site to the target site.

In still another embodiment of the invention, the immune entity can trigger the migration of the targeting cell towards the administration or pathological site.

In an embodiment of the invention, following the physico-chemical perturbation or the transport of the immune entity, there is a lapse of time of more or less than 10⁻⁶, 10⁻⁵, 10⁻³, 10⁻¹, or 1 second, 1, 15, 30, or 60 minutes, 2, 6, 12, or 24 hours, 3, 7, 15, or 30 days, 2, 3, 6, or 12 months, 2, 3, 6, or 10 years, between the activation by radiation and the migration of the targeting cell towards the administration or pathological site. This lapse of time can be that necessary for the targeting cell to detect, identify, recognize the physico-chemical perturbation and/or the immune entity and then be able to migrate towards the pathological or administration site.

In an embodiment of the invention, the targeting cell has targeted the administration or pathological site when it is located within the administration or pathological site respectively, or when it is located at a distance of less than 1 m, 10 cm, 1 cm, 1 mm, 100 µm, 10 µm, 1 µm, 100 nm or 10 nm from the administration or pathological site respectively.

In another embodiment of the invention, the targeting cell that targets the pathological or administration site also destroys the pathological cell or association and/or also removes the pathological cell or association from the target site. This preferentially occurs after the targeting cell has reached the pathological site. Preferably, the destruction or removal of the pathological cell or association can be activated more than 1, 2, 3, 4, 5, 6, 7, 8, 9, 10, 11, 12, 13, 14, 15, 20, 25, 30, 35, 40, 50, 75, 100, 150, or 200 times, preferentially by applying the radiation more than 1, 2, 3, 4, 5, 6, 7, 8, 9, 10, 11, 12, 13, 14, 15, 20, 25, 30, 35, 40, 50, 75, 100, 150, or 200 times, most preferentially by applying the radiation within the association, targeting or pathological site.

In an embodiment of the invention, the activation by radiation leads to the activation of the targeting cell, which may be involved in the destruction of the pathological cell.

In an embodiment of the invention, the activation by radiation leads to the inactivation of a targeting cell, which may be involved in the protection of the pathological cell.

In an embodiment of the invention, the activation by radiation leads to the activation of a receptor of the targeting cell. It can result in a change of activity, production, localization, expression of different types of receptors, such as molecular pattern recognition receptors (PRR), specific Toll-like receptor (TLR), NOD-like receptor (NLR), RIG-I-like receptor (RGR), or C-type lectin receptor (CLR), activated in the presence of patterns associated with pathogens (PAMP) and/or patterns associated with cell debris. These receptors that are expressed on a wide variety of immune cells such as natural killer (NK) cells, macrophages, dendritic cells, could be activated when the association is exposed to the radiation, producing cellular debris and/or an entity recognized by PRR. After activation, these receptors could participate in anti-tumor activity by promoting the capture of specific antigens. Antitumor activity could involve antigen presenting cells (APC) or the production of pro-inflammatory cytokines such as IL-6, IFN-β and TNF-β. Other receptors comprising antibodies, such as B cell receptor (BCR), may be activated by radiation. They may be able to recognize specific antigens originating from the association or pathological cell. T cell receptors (TCR), which are able to recognize specific MHC-antigen complexes, may also be activated by radiation.

In an embodiment of the invention, the activation by radiation may induce the activation of a targeting cell, which is an antigen presenting cell (APC). Within the environment of the targeting or pathological cell, APC, preferentially dendritic cells, could be activated by a signal originating from stressed targeting or pathological cells, capture the dead targeting or pathological cells or a part of these cells and induce a coupling between the specific tumor antigens and the major histocompatibility complexes such as MHC-1, MHC-2, or MHC-3. APC could then migrate to lymph nodes and activate B and/or T cells against the tumor. APC could also decrease the production of immunosuppressive cytokines such as TGF-β, IL-10, VEGF.

In an embodiment of the invention, the activation by radiation induces the activation of a targeting cell, which is a CD4+ T cell. The latter may be activated by antigens, preferentially specific tumor antigens. It may participate to the anti-tumor activity by secreting cytokines such as IL-2, which may stimulate B and/or T cells.

In an embodiment of the invention, the activation by radiation induces the activation of a targeting cell, which is a B cell. The latter may be activated by antigens, preferentially tumor antigens, and/or produce antibodies associated with these antigens, leading to anti-tumor activity.

In an embodiment of the invention, the activation by radiation results in the activation of a targeting cell involved in the immune antitumor activity, such as a killer cell, a T or a B cell, or results in the destruction of targeting cells having an immunosuppressive effect, such as Treg cells or myeloid cells.

In an embodiment of the invention, the activation by radiation can result in the activation of a targeting cell acting as an immune sentinel cell that maintains the integrity of organs and tissues, preferentially those infected by the pathological cell. The targeting cell can be activated after infiltration of the pathological cell or when there is a trauma. The targeting cell can then eliminate damaged healthy cells and initiate a cellular healthy cell repair process. This type of repair may not trigger any autoimmune response.

In an embodiment of the invention, the activation by radiation results in the activation of a targeting cell belonging to the innate or adaptive immune system after 0, 1, 15, 30, or 60 seconds, 2, 5, 15, 30, or 60 minutes, 2, 5, 10, or 24 hours, 2, 5, 10, or 30 days, 2, 6, or 12 months, 2, 5, or 10 years, following administration of the composition.

In an embodiment of the invention, the activation of the targeting cell belonging to the adaptive immune system can occur 0, 1, 15, 30, or 60 seconds, 2, 5, 15, 30, or 60 minutes, 2, 5, 10, or 24 hours, 2, 5, 10, or 30 days, 2, 6, or 12 months, 2, 5, or 10 years, after the activation of the targeting cell belonging to the innate immune system.

In an embodiment of the invention, the invention concerns a composition, in particular for use according to the invention, wherein the association is in suspension or in a solid matrix and is dispersed homogenously before it is brought into contact with the targeting cell and wherein the association, when brought into contact with the targeting cell, redistributes under activation by radiation either by concentrating at a site comprising the targeting cell or by being removed from a site comprising the targeting cell, and wherein the redistributed association destroys at least one pathological cell under activation by radiation.

According to the invention, the association can be dispersed homogenously before it is brought into contact with the targeting or pathological cell. It could happen before the composition is administered to an individual. For the association in liquid, it could mean that the absorption of the suspension, preferentially at a concentration in iron of less than 1000, 500, 100, 10, 1, or 0,1 mg per ml does not decrease by more than 90, 80, 70, 60, 50, 40, 30, 20, 10, 5, 4, 3, 2, or 1 %, where the absorption is measured at a wavelength, preferentially between 200 and 800 nm, more preferentially at 450 nm, where the variation in absorption is measured during 1, 10, 30, 60 or 120 minutes. For the association comprised within a volume, which is either in a solid matrix or in a gas, it could mean that the magnetic nanoparticle and/or the substance occupy or occupies more than 1, 10, 25, 50, 75, 80, 90, 95, or 99% of this volume.

According to the invention, the association can redistribute under activation by radiation by concentrating at a site comprising the pathological or targeting cell, where this concentration can mean that more than 10, 20, 30, 40, 50, 60, 70, 80, 90, or 95% of the association is located at the site comprising the pathological or targeting cell, where this percentage can represent the number of magnetic nanoparticles and/or substances located at the site comprising the pathological or targeting cells divided by the total number of magnetic nanoparticles and/or substances in an individual.

According to the invention, the targeting or pathological site can represent the volume containing at least two pathological or targeting cells respectively, which are separated by less than 3, 2, or 1 m, 1 cm, 100, 10, or 1 mm, 100, 10, or 1 µm, 100, or 10 nm.

According to the invention, the association can redistribute under activation by radiation by being removed from the pathological or target site, wherein this redistribution can mean that the magnetic nanoparticle and/or substance are 10⁹, 10⁸, 10⁷, 10⁶, 10⁵, 10⁴, 10³, 10², 10, 5, or 2 times less concentrated in the pathological or target site after than before activation by radiation.

In an embodiment of the invention, the invention concerns a composition or the composition comprising at least one association of at least one nanoparticle and at least one substance for use in a method of diagnosis of the presence of at least one pathological cell in an individual, comprising:
- firstly, administering the association at a body site of an individual wherein the presence of a least one pathological cell is suspected,
- secondly, activating the association, thereby leading to the migration of the targeting cell towards the association,
- thirdly, detecting the targeting cell, in order to reveal the presence of at least one pathological cell.

According to the invention, the association can be administered at the body site of an individual.

This could mean that the association is administered either directly at this site or far away from this site. If the association is administered far away from this site, it could then migrate to this site following administration.

According to the invention, the association can be activated by radiation and induce the migration of the targeting cell towards the association and/or towards the pathological cell.

According to the invention, the targeting cell can be detected, for example after a biopsy, by counting the number of pathological cells or the number of biological substances such as proteins, enzymes, DNA, RNA, receptors, or lipids, associated or linked to the pathological cell, by using histology, an assay, a spectroscopic or magnetic measurement method. The targeting cell may more easily be detected than the pathological cell due a more important number of targeting than pathological cell in the body site of an individual, or because of specific chemical or biological entities or mechanisms associated with the targeting cell that favor its detection.

In an embodiment, the composition for use according to the invention, the targeting cell under activation by radiation migrates towards the administration or pathological site.

In an embodiment of the invention, the targeting cell under activation by radiation receives, is targeted by, or is activated by the immune entity coming from the association or from the pathological cell. This can produce the attraction of the targeting cell by the pathological cell or by the association. This can also induce the migration of the targeting cell towards the pathological cell or towards the association.

In an embodiment of the invention, the targeting cell under activation by radiation is exposed to the physico-chemical perturbation or to a stress, which induces the attraction of the targeting cell by the pathological cell or by the association or the migration of the targeting cell towards the pathological cell or towards the association.

In an embodiment of the invention, the targeting cell is attracted by or migrates towards the association or pathological cell, when the targeting cell is separated by a distance of less than 1 m, 100 cm, 10 cm, 1 cm, 100 mm, 10 mm, 1 mm, 100 µm, 10 µm, or 1 µm from the association or from the pathological cell.

In an embodiment of the composition for use according to the invention, the targeting cell is a tumor cell or a cell infected by a pathogen.

According to the invention, a tumor cell can be a cell that does not divide at a regular rhythm or that divides more rapidly than a healthy cell. It can be a cell whose proliferation is inhibited.

It can be a cell that is programmed genetically to become a tumor cell, a cell that is becoming but is not yet a tumor cell. It can be a cancerous, a metastatic cell.

According to the invention, a cell infected by a pathogen can be a cell infected by at least one bacterium, preferentially a pathogenic bacterium, or one virus. It can be infected by bacteria or viruses that can't easily be destroyed by antibiotic or any type of available drug, in particular bacteria or virus that can resist drugs such as antibiotic but can be destroyed by radiations.

In an embodiment of the composition for use according to the invention, the administered association is concentrated at a site comprising a portion of the total number of pathological cell comprised in an individual at a concentration which is sufficient to enable activation by radiation.

According to the invention, the administered association can be concentrated at the site comprising a portion of the total number of pathological cell comprised in an individual. The site comprising a portion of the total number of pathological cell comprised in an individual can comprise more or less than 0.1%, 0.5%, 1%, 5%, 10%, 25%, 50%, 75%, 90%, or 95% of the total number of pathological cells comprised in an individual, where this percentage can represent the number of pathological cells at the site comprising a portion of the total number of pathological cells comprised in an individual divided by the total number of pathological cells in an individual. At the site comprising a portion of the total number of pathological cell comprised in an individual, the concentration in magnetic nanoparticles can be larger or lower than 10⁻⁹, 10⁻⁸, 10⁻⁷, 10⁻⁶, 10⁻⁵, 10⁻⁴, 10⁻³, 10⁻², 10⁻¹, 1, 10, 10², 10³, 10⁴, 10⁵, 10⁶, 10⁷, 10⁸, or 10⁹ mg in iron per mm³ or mm² or mm and/or the concentration in substances can be larger or lower than 10⁻⁹, 10⁻⁸, 10⁻⁷, 10⁻⁶, 10⁻⁵, 10⁻⁴, 10⁻³, 10⁻², 10⁻¹, 1, 10, 10², 10³, 10⁴, 10⁵, 10⁶, 10⁷, 10⁸ or 10⁹ mg of substances per mm³ or mm² or mm. The site comprising a portion of the total number of pathological cell comprised in an individual can also correspond to the volume comprising two pathological cells or more, wherein the pathological cells are separated by a distance of more or less than 1 m, 100 cm, 10 cm, 1 cm, 100 mm, 10 mm, 1 mm, 100 µm, 10 µm, 1 µm, or 100 nm.

Preferably, the site comprising a portion of the total number of pathological cell comprised in an individual can correspond to the cell site, the pathological site, the target site, the administration site, or the body site of an individual.

In an embodiment, the composition for use according to the invention, the administered association is redistributed by the targeting cell, by being concentrated at the site comprising a portion of the total number of pathological cell comprised in an individual or by being removed from this site.

According to the invention, the association can be captured, attached, or attracted by the targeting cell. The targeting cell can then concentrate the association at the site comprising a portion of the total number of pathological cell comprised in an individual at a concentration, which is 1, 2, 5, 10, 10², 10³, 10⁴, 10⁵, 10⁶, 10⁷, 10⁸, or 10⁹ lower or larger after than before the capture, attachment, or attraction of the association by the targeting cell.

In an embodiment, the composition for use according to the invention, at least one activation by radiation causes a temperature increase of more than 1 degree Celsius and/or another activation by radiation causes a temperature increase of less than 1 degree Celsius.

According to the invention, the association, preferentially the magnetic nanoparticle, can be concentrated at a concentration, preferentially in mg of iron per mm³, which is larger or lower than 0.01, 0.1, 1, 10, 100, 200, 500, or 1000 mg per mm³. The association can then be activated by radiation, producing a temperature increase larger or lower than 0.1, 1, 10, 25, 50, 75, 100, 500, 1000, or 10000 °C, where this temperature increase is preferentially measurable with a standard thermometry method.

In an embodiment of the composition for use according to the invention, the temperature is measured at a larger scale than the cellular scale.

According to the invention, the temperature can be measured at a larger scale than the micrometer scale, preferentially when the concentration of the suspension is sufficient to produce a temperature increase at such scale. Preferably, the temperature can also be measured at a smaller scale, such as the nanometer or nanoparticle scale, for example by attaching a molecule such as a fluorescent probe to the nanoparticle whose fluorescence varies with temperature and that can therefore be used as a nano-thermometer.

In another embodiment of the composition for use according to the invention, the activation by radiation is repeatable without readministration of the composition.

According to the invention, the composition can remain at the administration site, at the pathological or target site, or at the site comprising a portion of the total number of pathological cells comprised in an individual, at the association diffusion region, or at a site comprising more than 1, 10, 10², 10³, 10⁴, 10⁵, 10⁶, 10⁷, 10⁸, or 10⁹ pathological or targeting cells, preferentially more than 1, 2, 6, 12, 24, 48, or 72 hours, 4, 7, 15, or 30 days, 2, 6, or 12 months, 2, 5, or 10 years. This time can be long enough for the activation by radiation to be repeated more than 1, 2, 3, 4, 5, 6, 7, 8, 9, 10, 11, 12, 13, 14, 15, 20, 25, 30, 40, 50, 75, or 100 times without readministration of the composition.

In an embodiment of the invention, when the activation by radiation is repeated, it implies that the one or more of the mechanisms associated with this activation, which result in the weakening or destruction of the pathological cell, is/are also repeatable.

In another embodiment of the composition for use according to the invention, the activation by radiation specifically destroys the pathological cell without destroying healthy cells.

According to the invention, the activation by radiation can lead to an indirect mechanism of the pathological cell destruction, also designated in this invention as indirect mechanism.

In an embodiment of the invention, the activation by radiation results in the initial destruction of a portion of the total number of pathological cells comprised in an individual, or of less than 90%, 80%, 70%, 60%, 50%, 40%, 30%, 20%, 10%, 5%, 1%, or 0.1% of the total number of pathological cells comprised in an individual. After 1, 2, 5, 15, 30, or 60 seconds, 2, 15, or 60 minutes, 2, 4, 12, or 24 hours, 2, 15, or 30 days, 2, 6, or 12 months, 2, 10, or 20 years, following this initial destruction, the indirect mechanism occurs, which results in the destruction of another portion of the total number of pathological cells comprised in an individual, preferentially of the remaining portion of the total number of pathological cells comprised in an individual, or of more than 90%, 80%, 70%, 60%, 50%, 40%, 30%, 20%, 10%, 5%, 1%, or 0.1% of the total number of pathological cells comprised in an individual.

According to the invention, a portion of the total number of pathological cells comprised in an individual can represent more or less than 90%, 80%, 70%, 60%, 50%, 40%, 30%, 20%, 10%, 5%, 1%, or 0.1% of the total number of pathological cells comprised in an individual and is designated as the portion of pathological cells.

According to the invention, another portion of the total number of pathological cells comprised in an individual can represent more or less than 90%, 80%, 70%, 60%, 50%, 40%, 30%, 20%, 10%, 5%, 1%, or 0.1%, of the pathological cells that are not comprised in the portion of pathological cells. It is designated as the other portion of pathological cells.

In an embodiment of the invention, the indirect mechanism effect can occur following at least one other activation by radiation, which preferentially follows or is different from the initial activation by radiation.

In an embodiment of the invention, the activation by radiation is the initial activation by radiation or at least one other activation by radiation, which preferentially follows the initial activation by radiation.

According to the invention, the activation by radiation or the indirect mechanism specifically destroys the pathological cell without destroying healthy cells or with minimum damage to healthy cells. Such behavior, which may also correspond to the specific destruction of the pathological cell or the specific destruction of the pathological cell by the targeting cell, can prevent a disease or a life threatening condition just after, a few days, a few months, or a few years, following the treatment.

In an embodiment of the invention, the activation by radiation or the indirect mechanism stimulates or activates an immune entity, which results in the specific destruction of the pathological cell. An immune entity able to differentiate between a healthy and tumor cell can be a natural killer (NK) cell or a T cell, and can be activated by a receptor that promotes tumor cell destruction. The immune entity could destroy tumor cells by mobilizing for example lytic granules, cytokines, or interleukins.

In an embodiment of the invention, the activation by radiation activates the pathological cell that can be destroyed due to the absence at its surface of complex, ligand, or receptor, that prevent cell destruction such as the major histocompatibility complex of class I, while healthy cell could be spared due to the presence of such complex, ligand, or receptor, at its surface.

In another embodiment of the invention, the activation by radiation activates an immune entity, such as an interleukin, IL-2, IL-15, IL-12, IL-18, IL-21, IFN, a T cell, a dendritic cell, that could also be involved in promoting the survival, the proliferation, the toxicity, or the cytotoxicity, of the targeting cell, preferentially the toxicity or cytotoxicity of the targeting cell towards the pathological cell.

In an embodiment of the invention, the activation by radiation activates the targeting cell that destroys, preferentially specifically destroys, the pathological cell when it is an immune cell such as a CD4+ T or CD8+ cell. It can be activated by one or more other immune entities such as an interleukin. It can destroy the pathological cell by promoting inflammation, the production of an immune entity, a cytokine, a chemokine, or by recruiting neutrophils.

In an embodiment of the invention, the activation by radiation activates the targeting cell that destroys, preferentially specifically destroys, the pathological cell by producing: (i), cytotoxic chemicals, (ii), reactive oxygen species, nitrogen species, (iii), or inflammatory cytokines, such as IL-6. It may also destroy, preferentially specifically, the pathological cell by stimulating the activity of immune cells involved in pathological cell destruction such as T cells, granulocyte, neutrophils, or by reducing or stopping the activity of cells involved in the protection of proliferation of pathological cells such as Treg cells.

In another embodiment of the invention, the mechanism, preferentially the indirect mechanism, by which the pathological cell is selectively destroyed involves a cytotoxic substance, preferentially produced by a cell, which is more cytotoxic towards pathological than towards healthy cells.

In another embodiment of the invention, the mechanism, preferentially the indirect mechanism, by which the pathological cell is selectively destroyed involves a receptor, a ligand, a complex, which is activated or belongs to the pathological or targeting cell and which is not activated or does not belong to a healthy cell.

In another embodiment of the invention, the mechanism, preferentially the indirect mechanism, by which the pathological cell is selectively destroyed involves a receptor, a ligand, a complex, which is activated or belongs to a healthy cell and which is not activated or does not belong to a targeting or pathological cell.

In an embodiment of the invention, the mechanism, preferentially the indirect mechanism, by which pathological cells are selectively destroyed is due to a perturbation such as a variation in temperature, which is sufficient to produce pathological cell destruction and insufficient to induce healthy cell destruction. This can either be due to the fact that pathological cells are more sensitive to perturbation such as temperature variation than healthy cells or it can come from a higher concentration of the association in the pathological site than in the healthy site.

In another embodiment of the invention, the activation by radiation activates the targeting cell that destroys, preferentially specifically destroys, the pathological cell when the targeting cell is a dendritic killer cell, which can produce IFN-γ, NKT, or γδT cells.

According to the invention, the activation by radiation or the indirect mechanism can result in the activation of the targeting cell that specifically targets and destroys at least 1, 10, 10², 10³, 10⁴, 10⁵, or 10⁹ of pathological cells or the portion of pathological cells. The targeting cell is preferentially an immune entity that specifically targets and destroys pathological cells without targeting and destroying healthy cells.

According to the invention, the activation by radiation or indirect mechanism can result in the destruction of blood vessels, arteria, or any type of channel carrying blood, preferentially those supplying tumors with oxygen.

According to the invention, the activation by radiation can result in the apoptotic death of the pathological cell, preferentially of the portion of pathological cells. The indirect mechanism can then trigger or result in a signal of cell death, carried by heat, a chemical or a perturbation, which is transmitted from the pathological cell in an apoptotic state, initially activated by radiation, preferentially belonging to a portion of the total number of pathological cells, to the other pathological cell, not initially activated, preferentially belonging to the other portion of pathological cells. The pathological cell, not initially activated, preferentially belonging to the other portion of pathological cells, could then enter in a state of apoptosis.

According to the invention, the activation by radiation can result in necrotic cell death of the pathological cell, preferentially of the portion of pathological cells. The indirect mechanism can then be triggered by the necrotic pathological cells and stimulate an immune entity that specifically targets and destroys the pathological cell, preferentially the pathological cell belonging to the other portion of pathological cells.

In an embodiment of the composition for use according to the invention, wherein the activation by radiation leads to a majority of apoptotic or necrotic pathological cells.

According to the invention, more than 1%, 10%, 25%, 50%, 75%, 80%, 90%, or 95% of the total number of pathological cells comprised in an individual are in an apoptotic and/or necrotic state, where this percentage may represent the number of pathological cells in an apoptotic and/or necrotic state divided by the total number of the pathological cells in an individual. This percentage can be measured in an individual, at the body site of an individual, at different times following activation by radiation, just after radiation, 1, 2, 5, 10, 24, 48, 72, 150, or 200 hours following activation by radiation.

In another embodiment of the composition for use according to the invention, wherein the activation by radiation leads to a lower percentage of nanoparticles at the administration site.

According to the invention, the concentration in magnetic nanoparticles and/or substances at the target site decreases by more than a factor of 1.5, 2, 5, 10, 100, 10³, 10⁵, 10⁹ after activation by radiation, where this decrease can occur during the first 1, 2, 5, 10, 15, 30, or 60 minutes, 2, 6, 12, 24, 48, or 72 hours, 2, 5, 15, or 30 days, 2, 3, 6, or 12 months, 2, 5, or 10 years following activation by radiation. This decrease in concentration can be estimated by measuring the ratio between the concentrations of magnetic nanoparticles before and after activation by radiation.

In another embodiment of composition for use according to the invention, the nanoparticle is a magnetosome or a chemically synthesized nanoparticle with similar magnetic properties than a magnetosome.

According to the invention, the magnetic nanoparticle can be synthesized by a living organism, intracellularly or extracellularly, preferentially by a eukaryotic or prokaryotic cell, most preferentially by a bacterium such as a magnetotactic bacterium.

According to the invention, the magnetic nanoparticle can be considered synthesized by a living organism when one step in its fabrication process or in its use involves a contact or a processing by a living organism.

In an embodiment of the invention, a nanoparticle synthesized, at least in part, by a magnetotactic bacterium is called a magnetosome.

In an embodiment of the invention, the magnetotactic bacterium can be *Magnetospirillum magneticum* strain such as AMB-1, magnetotactic coccus strain such as MC-1, facultative anaerobic vibrios strains such as MV-1, MV-2 and MV-4, *Magnetospirillum magnetotacticum* strain MS-1, *Magnetospirillum gryphiswaldense* strain MSR-1, a facultative anerobic magnetotactic spirillum, such as *Magnetospirillum magneticum* strain MGT-1, an obligate anaerobe such as *Desulfovibrio magneticus* RS-1.

In an embodiment of the invention, the magnetic nanoparticle and/or the substance is/are nonpyrogenic. In this case, they preferentially contain less than 10000, 1000, 100, or 10 EU per mg of magnetic nanoparticle and/or per mg of substance.

In another embodiment of the invention, the magnetic nanoparticle and/or the substance contain a percentage of carbon, preferentially originating from the living organism synthesizing the magnetic nanoparticle, of less than 90, 80, 70, 60, 50, 25, 10, 5, 2, 1, or 0.5%. This percentage of carbon can be the weight or number of atoms of carbon contained in the magnetic nanoparticle and/or in the substance divided by the total weight or total number of atoms of the magnetic nanoparticle and/or the substance.

According to the invention, the magnetic nanoparticle is a chemically synthesized nanoparticle with at least one similar property than that of a nanoparticle synthesized by a living organism such as a magnetosomes, such as: (i), a coercivity, a remanent magnetization, a saturating magnetization, a specific absorption rate, which does not differ by more than a factor of 100, 10, 5, 2, or 1.5 from the coercivity, remanent magnetization, saturating magnetization and/or specific absorption rate of the magnetic nanoparticle synthesized by the living organism such as the magnetosome, (ii), a ferromagnetic or ferrimagnetic behavior, (iii), a high level of crystallinity, (iv), an arrangement in chain.

In an embodiment of the composition for use according to the invention, wherein the substance dissociates from the nanoparticle upon activation by radiation.

According to the invention, the substance dissociates from magnetic nanoparticles by activation by radiation and then activates the immune system.

In an embodiment of the composition for use according to the invention, wherein the radiation is selected from the group consisting of a magnetic field, a laser, an ionizing radiation and a sound radiation.

According to the invention, the radiation can be characterized by an intensity, a power, a strength or a frequency, which is constant, does not vary as a function of space or time, does not vary by more than 10, 25, 50, 75, 90, or 95% over a distance of more or less than 10 000, 1000, 100, 10, or 1 km, 100, 10, or 1 m, 10, or 1 cm, 1 mm, 100, 10, or 1 µm, 100, 10, or 1 nm, does not vary by more than 10, 25, 50, 75, 90, or 95% during a lapse of time of more or less than 10 000, 1000, 100, 10, or 1 year, 100, 10, or 1 day, 10, or 1 hour, 1 minute, 10, 1, 10⁻¹, 10⁻², 10⁻³, 10⁻⁶, 10⁻⁹, or 10⁻¹⁵ seconds.

According to the invention, the radiation can also be characterized by an intensity, a power, or a frequency, which varies as a function of time or space.

In an embodiment of the invention, the radiation is a magnetic field with a strength larger or lower than 1 µT, 10 µT, or 100 µT, 1 mT, 10 mT, or 100 mT, 1 T, 5 T, 10 T, or 100 T.

In an embodiment of the invention, the radiation is a magnetic field with a strength that varies spatially by more or less than 1 µT per mm, 10 µT per mm, or 100 µT per mm, 1 mT per mm, 10 mT per mm, or 100 mT per mm, 1 T per mm, 5 T per mm, 10 T per mm, or 100 T per mm. In an embodiment of the invention, the radiation is an oscillating or alternating magnetic field, a magnetic field with a strength that varies as a function of time at a frequency of more or less than 1 MHz, 1000 kHz, 100 kHz, 10 kHz, 1 kHz, 0.1 kHz, 0.01 kHz, or 0.001 kHz.

In an embodiment of the invention, the radiation has a power larger or lower than 0.01 W, 0.1 W, 1 W, 10 W, 100 W, 1000 W, 10000 W, 100000 W. It could be a light source with one or multiple wavelengths, a lamp or a laser. It could generate sufficient power to heat the magnetic nanoparticles.

In an embodiment of the invention, the radiation is an ionizing radiation, which may be able to generate free radicals.

In an embodiment of the invention, the radiation is an ultra-sound or a radiofrequency.

The present invention also relates to a composition comprising at least one association of two or more magnetic nanoparticles and a compound that stabilizes and/or coat the two or more magnetic nanoparticles and/or bind the two or more magnetic nanoparticles together, wherein the two or more magnetic nanoparticles without the compound possess a SAR of less than 200 W/gFe and wherein the association comprising the two or more magnetic nanoparticles and the compound possess a SAR larger than 200 W/gFe.

In an embodiment of the invention, the compound or the compound that stabilizes and/or coat the two or more magnetic nanoparticle and/or bind the two or magnetic nanoparticles together is the substance or the immune entity.

In an embodiment of the invention, the two or more magnetic nanoparticles comprise more than 2, 3, 4, 5, 6, 7, 8, 9, 10, 15, 20, 25, 30, 40, 50, 75, 100, 500, or 1000 magnetic nanoparticles. They can be the magnetic nanoparticle.

In another embodiment of the invention, the association of two or more magnetic nanoparticles and the compound possesses a specific absorption rate, SAR, which is lower or larger than 1, 10, 100, 200, 300, 400, 500, 600, 700, 800, 900, 10³, or 10⁴ Watt per gram of iron, W/gFe.

In still another embodiment of the invention, the two or more magnetic nanoparticles without the compound possess a specific absorption rate, SAR, which is lower or larger than 1, 10, 100, 200, 300, 400, 500, 600, 700, 800, 900, 10³, or 10⁴ W/gFe.

In still another embodiment of the invention, the association of two or more magnetic nanoparticles and the compound possess a SAR, which 1.0001, 1.001, 1.01, 1.1, 1.2, 1.5, 2, 5, 10, 100, or 1000 larger than the SAR of the two or more magnetic nanoparticles without the compound.

In still another embodiment of the invention, the SAR is measured In an or more of the following conditions: (i), at a concentration in magnetic nanoparticles that is larger than 0,1, 1, 5, 10, 20, 50, 100,200, or 400 mg of iron per mL of nanoparticle suspension, (ii), in an aqueous medium or in a gel, (iii), at a pH of more or less than 14, 13, 12, 11, 10, 9, 8, 7, 6, 5, 4, 3; 2, or 1 pH unit(s), (iv), at a temperature of more or less than 0,1, 1, 10, 50, 100, 200, 300, 400, 600, or 1000 K, (v), in a volume that is less than 1 liter, or 1 ml, or 100 µl, (vi), in a sufficiently small volume to avoid heat losses, (vii), in a sufficiently large volume to enable temperature measurements, (viii), in adiabatic conditions, or (ix), in conditions in which the suspension or matrix containing the two or more magnetic nanoparticles and possibly the compound is comprised within a tube or container that prevents heat losses of more than 100°C, 10°C, 1°C, 0,1, 0,01, or 0,001 °C per minute when the suspension is exposed to the alternating magnetic field.

In still another embodiment of the invention, the SAR is measured in conditions that enable to reach the maximum SAR.

According to the invention, the SAR can correspond to the maximum SAR.

According to the invention, the maximum SAR can be obtained by exposing various concentrations of the suspension or matrix containing the two or more magnetic nanoparticles and possibly the compound to an alternating magnetic field of fixed strength and frequency.

Above a certain concentration, called the saturating concentration, the SAR can remain constant and then correspond to the maximum SAR.

In an embodiment of the invention, the saturating concentration is larger or lower than 0.1, 1, 5, 10, 20, 50, 100, 200, or 400 mg of iron per mL.

According to the invention, the maximum SAR can also be obtained by exposing a fixed concentration of the suspension or matrix containing the two or magnetic nanoparticles and possibly the compound to an alternating magnetic field of varied strength and fixed frequency.

Above a certain field strength, called the saturating field strength, the SAR can remain constant and correspond to the maximum SAR.

In an embodiment of the invention, the saturating field strength is larger or lower than 0.1, 0.5, 1, 2, 5, 10, 20, 50, 100, or 500 mT.

According to the invention, the maximum SAR can also be obtained by exposing a fixed concentration of the suspension or matrix containing the two or magnetic nanoparticles and possibly the compound to an alternating magnetic field of fixed strength and varied frequency.

Above a certain field frequency, called the saturating field frequency, the SAR can remain constant and correspond to the maximum SAR.

In an embodiment of the invention, the saturating field frequency is larger or lower than 0.001, 0.01, 0.1, 1, 10, 20, 50, 100, 200, 500, 10³, 10⁴, 10⁵, or 10¹⁰ kHz.

In still another embodiment of the invention, the maximum SAR corresponds to the SAR measured above the saturating concentration, the saturating magnetic field strength, or the saturating magnetic field frequency.

In still another embodiment of the invention, high values of the maximum SAR, preferentially above 100, 200, 500, 700, 900, 1000 W/gFe are obtained by using one or more of the following methods: (i), by doping the magnetic nanoparticle with a transition metal such as cobalt, which may increase the magnetic anisotropy of the nanoparticle, (ii), by producing the magnetic nanoparticle with a specific geometry that enables to maximize the strength of its magnetic moment, (iii), by using a compound that improves the heating capacity of the two or magnetic nanoparticles, for example by preventing aggregation or by promoting rotation or uniform distribution.

In an embodiment of the composition for use according to the invention, the magnetic nanoparticle is synthetized by a living organism and the compound is not synthesized by this same living organism.

According to the invention, the magnetic nanoparticle is synthesized by the living organism when one or more of its fabrication steps, preferentially the majority or the most important of its fabrication steps, involves the living organism.

In an embodiment of the invention, fabrication steps of the magnetic nanoparticles can comprise: (i), the assembly of iron and possibly oxygen or other atoms to obtain a crystallized nanoparticle, (ii), the coating of the nanoparticle to stabilize the nanoparticle, (iii), the purification and/or sterilization of the nanoparticle to yield a low level of impurities and/or endotoxins in the nanoparticle or at the nanoparticle surface. The most important fabrication steps of the magnetic nanoparticle can correspond to the steps that lead to the magnetic properties of the nanoparticle.

According to the invention, the magnetic nanoparticle can be a metal, a metal oxide, or a mineral, synthesized by the living organism. The magnetic nanoparticle can contain less than 50, 40, 30, 20, 10, 5, 2, or 1% of carbon or of one or more atom(s) that do not activate under activation by radiation or that do not heat under the application of an alternating magnetic field, where carbon or one or more of this (these) atom(s) come from this organism or are produced by this organism. This percentage can represent the weight of carbon or of one or more of this (these) atom(s) comprised in the magnetic nanoparticle divided by the weight of the magnetic nanoparticle.

In an embodiment of the invention, the substance does not contain any material coming from or produced by the living organism producing the magnetic nanoparticle, or does not contain any material coming from or produced by another type of living organism. The substance can contain less than 50, 40, 30, 20, 10, 5, 2, or 1% of carbon or of one or more atom(s) that do not activate under activation by radiation or that do not heat under the application of an alternating magnetic field, where carbon or one of more of this (these) atom(s) come from this organism or are produced by this organism.

According to the invention, the composition is preferentially nonpyrogenic. It can then contain less than 10000, 1000, 100, 10, or 1 Endotoxin Unit per mg of nanoparticle.

In an embodiment of the invention, the invention also concerns the composition, wherein the magnetic nanoparticle is synthesized chemically and has the same coercivity and/or remanent magnetization and/or saturating magnetization and/or a ratio between remanent magnetization and saturating magnetization and/or SAR than the coercivity and/or remanent magnetization and/or saturating magnetization and/or ratio between remanent magnetization and saturating magnetization and/or SAR of the magnetic nanoparticle synthesized by the living organism, and wherein the compound is not synthesized by the living organism.

According to the invention, the magnetic nanoparticle is synthesized chemically when one or more of its fabrication steps, preferentially the majority or the most important of its fabrication steps, involves a chemical synthesis that preferentially does not involve a living organism.

Preferably, a living organism could be present during the chemical synthesis, for example if there is a contamination, but this living organism does not take part in the synthesis of the magnetic nanoparticle.

In an embodiment of the invention, the chemically synthesized magnetic nanoparticle is characterized by a coercivity and/or remanent magnetization and/or saturating magnetization and/or ratio between remanent magnetization and saturating magnetization and/or SAR, which differ(s) by a factor of less than 100, 10, 5, 2, 1.5, 1.2, or 1.1 from the coercivity and/or remanent magnetization and/or saturating magnetization and/or ratio between remanent magnetization and saturating magnetization and/or SAR of a magnetic nanoparticle synthesized by a living organism such as a magnetosome.

In another embodiment of the invention, the chemically synthesized magnetic nanoparticle is characterized by a coercivity and/or remanent magnetization and/or saturating magnetization and/or ratio between remanent magnetization and saturating magnetization and/or SAR, which is (are) equal to 100, 10, 5, 2, 1.5, 1.2, or 1.1 times the coercivity and/or remanent magnetization and/or saturating magnetization and/or ratio between remanent magnetization and saturating magnetization and/or SAR of a magnetic nanoparticle synthesized by a living organism such as a magnetosome.

In an embodiment of the invention, the invention concerns the composition, wherein the specific absorption rate of the association is larger than that of the magnetic nanoparticle synthesized by the living organism and surrounded by a coating substance, which contains organic material coming from the living organism.

In an embodiment of the invention, the SAR of the association is 1.01, 1.1, 2, 3, 5, 10, 100, or 1000 larger than that of magnetic nanoparticles synthesized by the living organism and surrounded by a coating substance, which contains organic material coming from the living organism.

According to this invention, the magnetic nanoparticle synthesized by a living organism and surrounded by a coating substance, which contains organic material coming from the living organism, can correspond to a chain of magnetosomes isolated from a magnetotactic bacterium.

It can be a chain isolated from the bacterium using a detergent such as NaOH, sonication, a French press or a pressure homogenizer. It is preferentially a chain of magnetosomes in which an organic membrane, made of lipids and proteins, surrounds the magnetosome crystallized and mineral core.

In an embodiment of the invention, the invention concerns the composition for use according to the invention to attract, detect, or destroy, a cell.

In an embodiment of the invention, the cell that is attracted, detected; or destroyed, is the immune entity, the pathological cell, or the targeting cell.

In an embodiment of the invention, the composition attracts the cell when the cell is moving or migrates towards the composition, preferentially at a speed of more or less than 1 meter per second, 1 meter per minute, 1 meter per hour, 1 meter per day, 1 meter per month, or 1 meter per year.

In another embodiment of the invention, the composition attracts the cell when the cell is located at a given time at a distance of less than any one of the following distances of 1 km, 100 m, 10 m, 1 m, 10 cm, 1 cm, 1 mm, 100 µm, 10 µm, 1 µm, 100 nm, or 10 nm, and preferentially when the composition is not located at this same distance at a the given time minus 10⁻⁹, 10⁻⁶, 10⁻³, 1, 10³, 10⁶, or 10⁹ seconds.

In still another embodiment of the invention, the composition detects the cell when it attracts the targeting cell and the targeting cell can be detected or when it destroys the pathological cell and the destroyed pathological cell can be detected.

According to the invention a cell can be detected either directly in the individual, or after it has been extracted from the individual.

In an embodiment of the invention, the composition destroys the cell either through the physico-chemical perturbation, preferentially through the physico-perturbation of the association, when the composition is activated by radiation.

In another embodiment of the invention, the composition destroys the cell by attracting the targeting cell towards the pathological site and the targeting cell then destroys the pathological cell, preferentially under activation by radiation.

In an embodiment of the composition for use according to the invention, the cell is a pathological or a targeting cell that specifically targets the administration or the pathological site.

### EXPERIMENTAL EXAMPLES:

### Signification of abbreviation.

In DX, X means the day following administration of U87-Luc cells in the mouse brains in examples 2, 5, 6 and 10 or the day following administration of glucose or the different suspensions of nanoparticles in GL-261 subcutaneous tumors in examples 8 and 9; MS designates a magnetic session; in MSX, X denotes the number of magnetic session; in MX, X designates mouse number, MC designates chains of magnetosomes isolated from AMB-1 magnetotactic bacteria; M-PLL designates the central part of magnetosomes or magnetosome minerals coated with poly-L-lysine; M-PNN designates MC or M-PLL co-localized with PNN, *i.e.* MC or M-PLL separated from PNN by a distance of less than 1 µm, AMF designates alternating magnetic field.

### References:

Part of the materials and methods presented in this invention were previously described in two patents: i), FR1501267 filed June 17, 2015 at INPI, France, entitled *"Nonpyrogenic preparation containing nanoparticles synthesized by magnetotactic bacteria for medical or cosmetic applications"* with PCT number PCT/FR2016/000095, filed June 15, 2016 at INPI, France, ii), FR1502228 filed October 21, 2015 at INPI, France, entitled *"Particle comprising at least one ferrimagnetic iron oxide nanoparticle associated with at least one compound for medical or cosmetic application*.*"*

### Example 1: Materials and Methods.

FR1501267 (page 18 to 19) describes methods of characterization of the different nanoparticles, in suspension or not. These methods enable: i), determination of the iron concentration of the different nanoparticle suspensions, ii), observation of nanoparticles by transmission electron microscopy (TEM), iii), determination of the endotoxin concentration of the different nanoparticle suspensions using the Limulus Amebocyte Lysate test (LAL), iv), estimates of the percentages of Carbon, Hydrogen, Nitrogen, Sulfur (CHNS) in nanoparticles, v), measurements by diffusion of nanoparticle zeta potentials and/or hydrodynamic sizes, vi), assessment of the suspension stability by absorption.

Nanoparticle characterization by Fourier transform infrared absorption method, FTIR, is described in International Journal of Pharmaceutics, Vol. 434, P. 444-452 (2012). Methods for measuring nanoparticle magnetic properties, including hysteresis cycle, ZFC and FC curves, are described in J. Phys. Chem C, V. 112, p. 12304 (2008). Nanoparticle specific absorption rate, SAR, measured in watts per gram of iron is estimated using the formula SAR=Cv(ΔT/δt)/C_{Fe}, where C_{V}=4.2 J/gK is the water specific heat capacity, (ΔT/δt), measured in °C per second, is the slope at the origin of the variation of temperature with time and C_{Fe}, measured in mg of iron per ml, is the nanoparticle concentration. Cytotoxicity of the various nanoparticles is estimated using an MTT assay following a protocol described in ACSNano, V. 5, P. 6279-6296 (2011). Preparation and characterization of suspensions containing magnetosome chains isolated from AMB-1 magnetotactic bacteria, designated as MC, are described in patent FR1501267 (page 21 and in Tables 1 and 2, pages 43 and 44). When a MC suspension is deposited on a substrate and observed by TEM, we observe that magnetosomes tend to align in a long chain, leading to a homogenous magnetosome distribution.

Magnetosomes have a cubo-octahedral geometry, a high level of crystallinity, a size that is larger than 20 nm for most magnetosomes, an average size of 45 nm, ferrimagnetic properties at physiological temperatures with a coercivity Hc of 200-300 Oe and a ratio between saturating magnetization and remnent magnetization Mr/Ms of 0.45. FTIR spectra, TEM and magnetic measurements of MC suggest that magnetosomes in MC are made of a core, composed of maghemite, surrounded by organic material that binds magnetosomes together in chains.

Endotoxin concentration of MC in suspension is estimated as 18000-150000 EU per mg in iron per ml of MC suspension, zeta potential of MC is estimated at -26 mV at pH 7. For 2 µl of a suspension containing 40 µg in iron of MC, mixed in water and exposed to an AMF of frequency 202 kHz and average strength 25 mT during 10 minutes, SAR of MC is estimated as 57 W/g_{Fe}. Synthesis and characterization of magnetosomes synthesized by AMB-1 magnetotactic bacteria cultivated in the presence of 400 µM rhodamine B and then extracted from these bacteria, designated as MCR400, are described in example 1 of patent FR1502228 (pages 32 to 34). Preparation and characterization of BNF-Starch suspension, purchased from Micromod (Micromod reference: 10-00-102), are described in example 1 of patent FR1501267 (pages 20 to 21). When a BN-Starch suspension is deposited on top of a carbon substrate and observed by TEM, we observe that BNF-Starch aggregate more than MC. BNF-Starch zeta potential is 10 mV at pH 7. They are surrounded by synthetic hydroxyethyl starch instead of biological material for MC. BNF-Starch core size without coating is estimated by TEM as 20 nm, leading to ferrimagnetic properties at physiological temperatures, but with lower values of Hc of 5.4 Oe than for MC. For 2 µl of a suspension containing 40 µg of BNF-Starch mixed in a water and exposed to the same AMF than with the MC, we measure a SAR of 10 W/g_{Fe}, lower than for the MC. Preparation and characterization of the central parts of magnetosomes isolated from MSR-1, designated by M, are described in example 3 of patent FR1501267, from pages 23 to 25 and in Tables 1 and 2. TEM measurements show that M tend to aggregate when they are deposited on a substrate and possess a small percentage in weight of carbon of 2.39%. M have a coercivity, Hc, of 10 mT, and are essentially composed of maghemite LAL tests show that suspensions containing M have an endotoxin concentration of 10-100 EU per mg in iron per ml of M suspension. For 1 mg of M mixed in an agar gel and exposed to the same AMF as for MC and BNF-Strach, we estimate a SAR of 128 W/g_{Fe}. Furthermore, M are unstable in suspension since the absorbance of 1 mg of M at 480 nm decreases rapidly by 80% in 20 minutes. Preparation and characterization of the central parts of magnetosomes isolated from MSR-1 magnetotactic bacteria, coated with poly-L-lysine, designated as M-PLL, are described in example 5 of patent FR1501267, pages 26 to 28 and in tables 1 and 2. Absorbance at 480 nm of 1 mg of M-PLL in suspension decreases by less than 20% in 20 minutes, indicating improved stability of M-PLL compared with M. For 1 mg of M-PLL mixed in an agar gel and exposed to the same AMF as for M and BNF-Strach, we estimated a M-PLL SAR of 61 W/g_{Fe}, a value, which is lower than that of M. Endotoxin concentration of a M-PLL suspension, measured by the LAL test, is 80 EU per mg of iron per ml of suspension. Cytotoxicity studies, pyrogenicity and acute toxicity of M-PLL are presented p. 28 to 30 of patent FR1501267. Preparation and characterization of the suspension containing the central parts of magnetosomes isolated from MSR-1, coated with polyethyleneimine, M-PEI, are described in example 15 of patent FR1501267, page 40. Protocol used for cell preparation to carry out *in vivo* experiments are described for MDA-MB231 cells in ACSNano, V. 5, p 6279-6296 (2011). Similar protocols are used for preparing U87-Luc and GL261 cells. In the different mouse treatments, mice are treated, fed and watered following ethical rules. Mice are euthanized by cervical dislocation when weight losses exceed 20%, or when signs of pain, unusual posture or prostration are observed or when tumor sizes are larger than 1000-2000 mm³. In examples 2, 5 and 6, a cell suspension containing 10⁵ U87-Luc cells per microliter is inoculated in brains of nude CD-1 female mice of 20 g, 7 weeks old, purchased from Charles River. At day 0, D0, mice are anesthetized with a mixture of ketamine/xylazine. Mouse head is positioned in a stereotactic frame, a craniotomy is performed at fixed coordinates (0.2.0) and 2 µl of a suspension containing 2.10⁵ U87-Luc cells are administered at (0.2.2). In examples 2 and 5, tumors grow during 8 days and nanoparticle or glucose administration is carried out at D8 while in example 6, tumors grow during 5 days and nanoparticle or glucose administration is carried out at D5. In examples 2, 5 and 6, tumor surface temperature is measured over time during the various MS using an infrared camera (EasIRTM-2, Optophase) placed 20 cm above the coil. We measure using a thermocouple (IT-18, Physitemp, Clifton, USA) the temperature at the tumor cell administration site, which can be associated with the pathological site, in a dead mouse receiving 40 µg of MC or M-PLL. We find that this temperature is similar to that measured with the infrared camera, suggesting that during the various MS, surface temperature measured with the infrared camera is similar to the temperature at the tumor cell administration site. In examples 2, 5 and 6, to follow variation of tumor volume with time, tumor bioluminescence intensity (BLI) is measured using an IVIS Spectrum 10 minutes after intraperitoneal administration into each mouse of 2 mg of luciferin mixed with 100 µl of PBS and a relation between tumor BLI and tumor volume is determined. Survival times of the different mouse groups are expressed as averages ± standard deviations, with indications of p-value estimates.

Histological studies are carried out on brain sections of mice treated similarly to those of examples 2, 6 and 10 fixed with a 4% solution of paraformaldehyde, cut into transverse slices of 2 mm thick and embedded in paraffin. Sections of paraffin blocks, 4 µm thick, are deposited on glass slides and stained with hematoxylin-eosin (H&E) and/or Prussian blue to distinguish between healthy and tumor sites and to determine magnetosome location, polynuclearneutrophiles (PNN), heathy and tumor cells. In examples 2 and 6, the different sections studied by histology are designated as HMC6h and HMC72h for sections collected from mice euthanized 6 hours and 72 hours following intratumor administration of 40 µg of MC respectively, HMC0hB for those collected from mice receiving 40 µg of MC and euthanized just after one MS, HMC4hB for those collected from mice receiving 40 µg of MC and euthanized 4 hours following a first MS, HMC12hB for those collected from mice receiving 40 µg of MC and euthanized 12 hours following a first MS, HMC24h3B for those collected from mice receiving 40 µg of MC and euthanized 24 hours following 3 MS, HMPLL6h and HMPLL72h for those collected from mice euthanized 6 hours and 72 hours after intratumor administration of 500 µg of M-PLL, HMPLL5.5hB for those collected from mice receiving M-PLL and euthanized 5 hours and an half following a first MS, HMPLL24h3B for those collected from mice receiving 500 µg of M-PLL and euthanized 24 hours following a third MS, HBNF6h and HBNF72h for those collected from mice euthanized 6 hours and 72 hours following intratumor administration of 500 µg of BNF-Starch respectively, HBNF5.5hB for those collected from mice receiving 500 µg of BNF-Starch euthanized 5 hours and an half following a first MS, HBNF24h3B for those collected from mice receiving 500 µg of BNF-Starch euthanized 24 hours following a third MS.

### Example 2: Magnetosomes chains extracted from AMB-1 magnetotactic bacteria introduced into U87-Luc brain tumors and exposed to an AMF lead to full tumor disappearance.

At D8, 7 different groups of mice receive at the coordinates (0.2.2) 2 µl of the various solutions or suspensions containing either 5% glucose (groups 1 and 2), 40 µg in maghemite of MC (groups 3, 4, 5) or 40 µg in maghemite of BNF-Starch (groups 6 and 7).

Groups 1, 3 and 6 do not undergo any further treatment after D8. After D8, groups 2 and 7 are exposed to 12 MS at D8 (MS1), D9 (MS2), D10 (MS3), D15 (MS4), D16 (MS5), D17 (MS6), D22 (MS7), D23 (MS8), D24 (MS9), D29 (MS10), D30 (MS11) and D31 (MS12). Groups 4 and 5 are exposed to MS1 to MS12 and to additional MS at D36 (MS13), D37 (MS14) and D38 (MS15). During each MS mentioned in this example, mice are exposed to an AMF of average strength 25 mT and frequency of 202 kHz during 30 minutes. For the different groups of mice, BLI is measured at D7, D14, D21, D28, D35, D42, D49 and D56. Untreated mice (group 1), mice exposed to 15 MS (group 2), mice receiving at D8 40 µg of MC (group 3) or 40 µg of BNF-Starch (group 6) are prone to an increase of their tumor volume after tumor cell implantation at D0. The average tumor volume increases from 30 mm³ at D7 to 40 mm³ at D28 (group 1), from 10 mm³ at D7 to 160 mm³ at D35 (group 2), from 5 mm³ at D7 to 265 mm³ at D35 (group 3), from 15 mm³ at D7 to 60 mm³ at D28 (group 6). Average tumor temperature of these mice, measured during the different treatments, do not vary. In addition, mice belonging to these groups are rapidly euthanized, between D28 and D42, due to weight losses exceeding 20%. Average survival time is also low and similar for these mice at 40.3 ± 1.1 days (group 1), 39.4 ± 2.3 days, p=0.938, (group 2), 46.4 ± 2.3 days, p=0.028, (group 3), 36.8 ± 1.1 days, p=0.053, (group 6). Signs of antitumor activity aren't observed among these mice. By contrast, for mice belonging to group 5, receiving 40 µg of MC in U87-Luc intracranial glioblastoma tumors of average volumes ∼ 25 mm³ followed by 15 MS, mean tumor volumes decrease first from 25 mm³ at D7 down to 5 mm³ at D14, then increase from 5 mm³ at D14 to 70 mm³ at D42. During the various MS, brain tumor temperatures increase by an average of 4°C at D8 (MS1), 1.7 °C at D9 (MS2), 0.4 °C at D10, D15 and D16 (MS3 to MS5), and do not increase after D17.

For a mouse with a typical behavior belonging to group 5, the variation over time of the tumor volume shows a series of oscillations, a decrease from 7 mm³ at D7 to 2 mm³ at D14, between MS1 and MS3, an increase from 2 mm³ at D14 to 9 mm³ at D21, between MS4 and MS6, followed by a decrease from 9 mm³ at D21 to 2 mm³ at D28, between MS7 and MS9, followed by an increase from 2 mm³ at D28 to 9 mm³ at D35, between MS10 and MS12, followed by a decrease from 9 mm³ at D35 to 2 mm³ at D42, between MS 13 and MS 15, followed by an increase from 2 mm³ at D42 to more than 14 mm³ at D45. These oscillations suggest that the application of the AMF leads to clear and repetitive anti-tumor activity. MS 15 does not however prevent tumor regrowth. For mice belonging to group 5, anti-tumor efficacy does not seem sufficient to increase mouse survival time, which is of 46.5 ± 1.7 days, p = 0.015, similar to that of mice belonging to groups 1, 2, 3 and 6. The presence of a tumor of large-size in mice belonging to group 5 may require the administration of more than 40 µg of magnetosomes to lead to complete tumor disappearance. Mice belonging to group 4 are treated similarly to those of group 5, except that average tumor volumes are smaller at 1 mm³ at D7. In mice belonging to group 4, tumor gradually disappears following MS1 to MS15, leading to the complete tumor disappearance without tumor regrowth at D35. For a typical mouse belonging to group 4, the tumor volume decreases from 3 mm³ at D7 to 1 mm³ at D28 to 0 mm³ at D 42. Temperature variations during the different treatments are similar to those observed in group 5. For these mice, a mean survival time of 83.9 ± 4.3 days (p <0.0001) is estimated, which is 42 days on average longer than that of the other groups. This high value is however an underestimate since mice are euthanized at D150 to carry out brain histological analysis. 40% of mice belonging to group 4 are still alive at D150. At D150, mice, which are still alive, are euthanized and slices of their brain are imaged by optical microscopy. These images indicate the absence of tumor or lesion, which suggests that these mice are completely cured. Mice receiving at the center of intracranial glioblastoma tumors 40 µg of BNF-Starch followed by 15 MS (group 7) behave differently from those belonging to groups 4 and 5. Indeed, tumor volumes of these mice increase continuously from 3 mm³ at D7 to 60 mm³ at D37. In addition, in group 7, animal mean survival time is estimated as 45.5 ± 2.3 days (p = 0.053), a similar value to that estimated for groups 1, 2, 3, 5 and 6, suggesting that intratumor administration of BNF-Starch, followed by multiple MS, does not trigger any anti-tumor efficacy. A histological study is carried out on animals treated similarly to those belonging to groups 1 to 7 and euthanized at various times after treatment. In mice treated similarly to those of groups 1 and 2, receiving glucose at D8, followed (or not) by MS, which are euthanized 6 or 24 hours following glucose administration or 6 hours after one MS or 24 hours after a third MS, optical microscopy images of histological sections of mouse brains show a well-defined tumor with an elliptical shape, located at one site without any sign of damage that could have resulted from glucose administration. Furthermore, the presence of polynuclear neutrophils (PNN), also called granulocytes, is not observed in these images. The same type of tumor is observed in mice treated in the same way as those of group 3, receiving at D8 2 µl of a suspension containing 40 µg of MC, euthanized 6 or 72 hours after MC administration. HMC6h and HMC72h show that approximately 50% of the tumor surface is brown, suggesting the presence of MC at this site. At 6 hours (HMC6h), magnetosomes and PNN are not separated by more than 1 µm, are therefore located at the same site and co-localized magnetosomes and PNN are designated as M-PNN. PNN alone or magnetosomes alone are not observed. 8% of M-PNN are observed in the ventricles, suggesting that M-PNN could carry MC outside of the tumor through the blood vessels of the ventricles. 92% of M-PNN are observed in the tumor or in its periphery. We estimate that there are 18413 M-PNN on average per mm² of tumor. At 72 hours (HMC72h), magnetosomes seem more scattered than at 6 hours. There aren't any M-PNN or PNN alone, suggesting that PNN disappear between 6 and 72 hours following MC administration. In these mice, necrosis or apoptosis is not observed. For mice treated similarly to those belonging to groups 4 and 5, receiving 2 µl of a MC suspension at D8, euthanized immediately after the first MS (HMC0hB), we observe that magnetosomes occupy only 8% of the tumor surface, that they are predominantly located at the tumor periphery and that the amount of M-PNN is 4800 M-PNN on average per mm² of tumor. 4 hours after the first MS (HMC4hB), PNN alone or magnetosomes alone aren't observed and there are approximately 12800 M-PNN per mm² of tumor. Half of M-PNN occupy 15% of the tumor. The other half is observed at the tumor periphery. Considering mice euthanized 12 hours after a first MS (HMC12hB), just after two MS (HMC0h2B), or 24 hours after three MS (HMC24h3B), about 90% of magnetosome are located near or in the PNN, 10% of them are near or in U97-Luc tumor cells and PNN are observed near the M-PNN area, which suggests that PNN are attracted by the magnetosomes.

A fraction of M-PNN occupies about 7% of the tumor area, while another M-PNN fraction is localized in the ventricles. We observe approximately 27852 M-PNN per mm² of tumor. In HMC24h3B, there are 5 areas containing U87-Luc cells of much smaller surfaces than tumor surfaces observed just after or 4 hours after a first MS, suggesting that after three MS, treatment acted, causing a splitting of the initial tumor area, located at one site into smaller tumor surfaces located at different sites. This antitumor behavior in HMC24hB is further characterized by the presence of sites containing U87-Luc cells in a state of necrosis or apoptosis with magnetosomes internalized in U87-Luc cells or located at the surface of these cells, *i.e.* magnetosomes are mainly not separated by more than 1 µm from U87-Luc cells. In HMC24h3B, magnetosomes are either located near or in PNN or near or in U-87-Luc cells, *i.e.* magnetosomes are mainly not separated by more than 1 µm from PNN or U87-Luc cells. They are also located near the ventricles and small tumor portions, *i.e.* magnetosomes are mainly not separated by more than 1 µm from ventricles and small tumor portions. Compared with mice receiving MC with or without MS, those treated similarly to groups 6 and 7, receiving 2 µl of a suspension containing 40 µg in iron of BNF-Starch and euthanized 6 hours after BNF-Starch administration, or receiving the same BNF-Starch suspension and euthanized 24 hours after a third MS display a significantly different behavior. Indeed, on the one hand, PNN are not observed in histological images of brain sections. On the other hand, histological images show a large and well defined tumor without necrosis, suggesting that application of three MS in the presence of BNF-Starch into the tumor does not induce any antitumor activity. Pharmacological antitumor effect due to MC or BNF-Starch cytotoxicity in the absence of heat does not seem to occur. Administration of MC or BNF-Starch without AMF application (groups 3 and 6) does not appear to induce any anti-tumor efficacy. Secondly, anti-tumor efficacy may be due to cell mechanical disruption under application of an AMF without heat production. The AMF may induce a magnetosome movement, which weakens cells or cell membranes, leading to their destruction. In the total absence of heat, such a mechanism is however unlikely to occur since mice belonging to group 7, receiving BNF-Starch and exposed to 15 MS without any increase in tumor temperature, do not display any decrease in tumor volume following MS. However, for mice belonging to groups 4 and 5, in which the tumors are first heated during 4 MS and then unheated during the 11 remaining MS, antitumor activity is observed during the 11 remaining MS. This suggests that when tumor tissue is first sensitized by heat, anti-tumor activity may be triggered by the application of an AMF, which does not produce any temperature increase. It should be noted that the probes that we use to detect the temperature is not sensitive enough to detect temperature increase at cellular level or below (organelle, cell seed, individual nanoparticle...). It is therefore possible that temperature increase occurs at small scale, but it is not detected. Third, the mechanism responsible for tumor destruction involves the heat produced by MC under application of an AMF since the only groups of mice which display clear anti-tumor efficacy are groups 4 and 5 whose tumors are heated. Moreover, in example 6, we show that increasing the amount of heat produced within the tumor by administering a larger amount of 500 µg of magnetosomes instead of 40 µg in this example leads to a percentage of mice, which are fully cured of 100% instead of 40% in this example. This supports the idea that heat is necessary to produce tumor destruction. When tumor tissues are heated to 40-45 °C during 30 minutes, as in mice belonging to groups 4 and 5 during the first 4 MS, irreversible cell damage could occur, which may be caused by: i), a change in cell membrane integrity, fluidity or permeability, ii), cytolysis, iii), dysfunction of actin filaments, microtubules or mitochondria. Cell damage could also be associated with the inhibition of the replication of DNA or RNA synthesis, to denaturation of the DNA polymerase. Hyperthermia may also lead to inflammatory infiltrates near necrotic areas with the presence of several immune cells such as dendritic cells, natural killer cells, as well as B and T cells. In this example, we find that cells of the immune system, called polynuclear neutrophils (PNN), are located at the same site as the magnetosomes for a mouse euthanized 6 hours after MC administration without MS, euthanized 4 hours after one MS or 24 hours after three MS. However, PNN are not observed after administration of MC followed by MS or 72 hours after MC administration without MS. These results suggest that PNN gradually migrate in the site, where magnetosomes are located between 0 and 6 hours following MC administration. Later on, PNN leave this site or are destroyed in the absence of application of the AMF. In fact, it is likely that the organic material surrounding the inorganic magnetosome core, which is mainly composed of lipids, proteins and LPS, is responsible for the recruitment of PNN. Indeed, when BNF-Starch with similar iron oxide composition than the magnetosome crystallized core but devoid of any organic bacterial residues are administered to tumors, neutrophils are not observed in tumors. In fact, PNN located at the same site as the magnetosomes, called M-PNN, are observed during a longer period of time in the presence than in the absence of MS. The application of AMF could dissociate the organic material from the magnetosome mineral core, leading to the attraction and migration of neutrophils towards the magnetosomes. Furthermore, the presence of M-PNN next to the ventricles after treatment suggests that PNN may carry magnetosomes outside of the tumor through the ventricle blood vessels. This behavior agrees with the known neutrophil function, which is to get rid of organic substances of bacterial origin. Since PNN are observed in the tumor site and the mice used in this study have Toll-like receptors, they could be involved in the tumor destruction, a type of behavior that has previously been observed in mice bearing B 16 tumors. However, this assumption is uncertain since 100% of mice bearing the same U87-Luc tumors and treated similarly than those of groups 4 and 5, but with less immunogenic magnetosomes containing an amount of endotoxins of 80 EU per mg per ml and 5% of organic carbon at the magnetosome surface, compared with 18 000-150 000 EU per mg per ml and 14% of organic carbon for the MC are completely cured despite of a lower number of PNN (example 6). This suggests a greater efficacy with less immunogenic magnetosomes and a lower amount of PNN. In mice belonging to example 6, tumors are heated to higher temperatures during longer times, suggesting that, if the immune system is involved in the destruction of the tumor, its antitumor activity may come from heat and not from the intrinsic magnetosome immunogenicity. Histological analysis suggests that magnetosomes only occupy a small part of the tumor surface after the first MS (7-15%). This suggests that the destruction of a small tumor portion by magnetosomes exposed to AMF may induce the destruction of the remaining tumor portions. This mechanism could be of immunological origin as described above, apoptotic since heat is known to promote apoptosis and death of tumor cells through this mechanism, or be the result of damages by heat to the blood vessels irrigating the tumor, a behavior which may occur in mice of groups 4 and 5 since magnetosomes are observed in the ventricles, near blood vessels.

Previous experiments, carried out with 2-3 mg of smaller superparamagnetic iron oxide nanoparticles (SPION) of 15 nm, administered in RG-2 or T-9 tumors produces tumor heating to 43-47 °C during 30 minutes by applying an AMF. This treatment resulted in a 15 to 44-day survival time following tumor implantation compared with 8 to 14 days for untreated rats. Due to better magnetosomes heating properties compared with SPION, the amount of magnetosomes necessary to eradicate U87-Luc tumor in this example is 20 µg per mm³ of tumor, lower than 60 µg per mm³ of tumor used to produce an even less efficient anti-tumor activity in previous studies. We can conclude from this example that: i) it is possible to completely eliminate U87-Luc glioblastoma tumors implanted in the brain of mice by intratumoral administration of 40 µg in iron oxide of MC, followed by 15 MS during which a AMF frequency of 202 kHz and average strength 25 mT is applied, ii), the antitumor activity is probably not due to the MC cytotoxicity, iii), antitumor activity is triggered by the heat produced by MC as BNF-Starch administered at the same quantity as MC and exposed to the same MS do not produce any tumor temperature increase and do not induce any anti-tumor activity, iv) in the presence of MC, it is possible to induce anti-tumor activity by applying an AMF, which doesn't produce any temperature increase provided that the tumor has been heated beforehand, v), the more the tumor is heated, the higher the anti-tumor activity is (comparison between examples 2 and 6), vi), it is possible to have immune cells (neutrophils) migrate towards MC, expected to be due to the presence of organic material at magnetosome surface, where this migration is enhanced by the application of an AMF, vii), since the tumor can be completely destroyed while magnetosomes only occupy a small percentage of tumor surface, it is possible that indirect mechanisms are responsible (at least in part) for tumor destruction, such as immunogenic, apoptotic or due to the destruction of the blood vessels irrigating the tumor.

In such indirect mechanism, tumor cells containing nanoparticles could induce the destruction of the other tumor cells not containing the nanoparticles.

### Example 3: In vitro treatment of U87-Luc cells brought into contact (or not) with MC and exposed (or not) to a MS.

A suspension containing 40 µg in maghemite of MC is brought into contact with 10³, 5.10³, 10⁴, 5.10⁴, 10⁵ or 5.10⁵ U86-Luc cells and exposed (or not) to a MS during which an AMF of average strength 25 mT and frequency 202 kHz is applied during 30 minutes. The mixture is then incubated during 4 hours at 37 °C in the presence of 5% CO₂. 10 minutes before the end of incubation, 2 µl of luciferin at 20 mg/ml are added to the mixture producing luminescence of living cells whose BLI is measured using an IVIS spectrum. For cells alone, BLI of U87-Luc cells increases linearly with the number of cells from 0 a.u. in the absence of cells up to 1.5 10⁹ a.u. in the presence of 5 10⁵ cells. When U87-Luc cells are brought into contact with MC in the presence (or not) of a MS, BLI first increases linearly with the number of cells up to 10⁵ cells, then saturates beyond 10⁵ cells. BLI saturation may be due to MC that promote cell death. The linear coefficient of BLI increase with increasing cell number decreases from 2500 for cells alone down to 210 for cells brought into contact with MC and down to 7 for cells brought into contact with MC in the presence of a MS. From the values of these linear coefficients, we deduce that the number of living cells decreases from 100% for cells alone to 8.4% for cells brought into contact with MC and 0.3% for cells brought into contact with MC in the presence of a MS. BLI saturation is probably not due to magnetosome screening, since such screening should prevail when the ratio between the number of magnetosomes and the number of cells is the highest, *i.e.* at low cell concentration. In addition, there would be no reason why such effect would be enhanced in the presence of the AMF since magnetosome concentration remains the same in the presence (or not) of the AMF. Instead, this loss of linearity could be associated with cells being destroyed by MC in the presence (or not) of a MS inducing the death of other cells not directly destroyed by MC. An indirect mechanism of cell death could occur whereby cells destroyed by MC with or without MS emit a signal or release a toxin that induces the death of others cells not initially destroyed by MC.

### Example 4: In vitro determination of the type of cellular death (apoptosis or necrosis) when U87-Luc cells are brought into contact (or not) with MC and exposed (or not) to a MS.

U87-Luc cells are cultivated in 30 mm Petri dishes at a density of 500000 cells per dish and brought into contact with: a 5% glucose solution (treatment 1), a M-PLL suspension containing 1 mg of iron in 1 ml (treatment 2), a M-PLL suspension containing 1 mg of iron In an ml followed by a MS which maintains the temperature at 45 °C during 30 minutes (treatment 3). 12 hours following treatments, cells are washed twice with PBS, are harvested by trypsinization and are incubated with Annexin V and propidium iodide for apoptosis and necrosis detection respectively. Invitrogen kit V13241 is used. Using a flow cytometer, luminescence of the treated cells is excited at 488 nm and luminescence intensity is detected at 530 nm and 575 nm for apoptosis and necrosis detection respectively. Treatments lead to 99% of living cells and 1% of necrotic cells (treatment 1), to 40% of living cells, 34% of necrotic cells and 26% of apoptotic cells (treatment 2), to 10% of living cells, 27% of necrotic cells and 63% of apoptotic cells (treatment 3).

### Example 5: Treatment of intracranial U-87 Luc tumors by administration to these tumors of 40 µg in iron oxide of MC followed by 3 MS during which an AMF of average strength 25 mT and frequency 198 kHz is applied during 30 minutes.

A group of 10 mice is treated similarly to group 4 of example 2, except that mice are exposed to three MS at D8, D9 and D10 instead of fifteen MS for group 4. For mice exposed to 3 MS, tumor volumes increase continuously following the three MS and average survival time is 41 ± 1 days, similar to the average survival time observed among untreated mice. For 3 MS anti-tumor activity is not observed and it is therefore necessary to use more than 3 MS to remove the tumor.

### Example 6: Full disappearance of intracranial U-87 Luc tumors by intratumor administration of M-PLL followed by AMF application.

At D5, 6 different groups, containing 9 mice each, receive at the site of tumor cell implantation, 2 µl of different solutions or suspensions containing either 5% glucose (groups 1 and 2), 500 µg in iron of M-PLL (groups 5 and 6) or 500 µg in iron of BNF-Starch (groups 3 and 4). Groups 1, 3 and 5 do not undergo any further treatment. After D5, groups 2, 4 and 6 are exposed to 15 MS at D5, D6, D7, D12, D13, D14, D19, D20, D21, D26, D27, D28, D33, D34 and D35 for group 2, 20 MS at D5, D6, D7, D12, D13, D14, D19, D20, D21, D26, D27, D28, D33, D34, D35, D40, D41, D42, D47 and D48 for group 4, to 27 MS at D5, D6, D7, D12, D13, D14, D19, D20, D21, D26, D27, D28, D33, D34, D35, D40, D41, D42, D47, D48, D49, D54, D55, D56, D61, D62 and D63 for group 6. Each MS involves the application of an AMF of frequency 202 kHz and average strength 25 mT applied during 30 minutes. 4 mice of group 6 and 5 mice of group 4 receive a second administration of 200 µg in iron of M-PLL (group 6) and 200 µg in iron of BNF-Starch (group 4) at D47 due to tumor regrowth. Intracranial administration of suspensions containing M-PLL or BNF-Starch in glioblastoma tumors, followed or not by MS, is safe, since signs of toxicity are not observed in animals receiving these treatments. Untreated mice (group 1) and mice exposed to 15 MS (group 2) are prone to tumor volume increase following D0. The average tumor volume increases from 2 mm³ at D0 to 200 mm³ at D45 (group 1) and from 2 mm³ at D0 to 180 mm³ at D45 (group 2). Average brain tumor temperatures of these mice, measured during these MS, do not vary. In addition, mice belonging to these groups are rapidly euthanized, between D28 and D45, due to weight losses exceeding 20%. Average survival time is also quite low and similar for these mice at 44 ± 2 days for group 1 and 44 ± 1 days (p = 0.662) for group 2. Signs of anti-tumor activity are not observed among these mice. For mice belonging to group 5, which receive M-PLL in intracranial U87-Luc tumors without AMF application, average tumor volume increases less rapidly than in mice of groups 1 and 2. This delay in tumor growth results in an average survival time of 114±7 days in these mice, which is higher than that of groups 1 and 2, indicating anti-tumor activity, which may be attributed to poly-L-lysine cytotoxicity. After having received the same suspension of M-PLL than mice belonging to group 5, mice of group 6 are exposed to MS. For these mice, average tumor volumes rapidly decrease from 1.5 mm³ at D0 to 0 mm³ at D42, resulting in total tumor disappearance in five mice without tumor regrowth or a decrease in tumor volumes from 1.5 mm³ at D4 to 0.6 mm³ at D30, then a tumor volume increase from 0.6 mm³ at D30 to 1.5 mm³ at D47 in 4 mice.

Because of tumor regrowth in these four mice, M-PLL suspension was administered a second time at D47. After this second administration followed by 9 MS, tumor volume decreases from 1.5 mm³ at D47 to 0 mm³ at D68 and the tumor completely disappears in all mice belonging to group 6 at D68. After D4, tumor volume either decreases quickly to reach 0 mm³ at D11 for a typical mouse or first decreases to 0.8 mm³ at D11, then increases from 0.8 mm³ at D11 to 1.6 mm³ at D45 and then decreases again from 1.6 mm³ at D45 to 0 mm³ at D69 for another typical mouse. Average tumor temperature increases are 17.5 °C at D5 (MS1), 16.5 °C at D6 (MS2), 10 °C at D7 (MS3), 7 °C at D12, D13 , D14, D19, D20, D21, D26, D27, D28, D33, D34 and D35 (MS4 to MS 15), 2 °C at D40 (MS16). It then remains unchanged at D41, D42, D47, D48, D49, D54, D55, D56, D61, D62, D63 (MS17 to MS27) for M46, M48, M49, M50 and M51 or remains unchanged at D41 and D42 (MS 17 and MS 18), and increases as a result of M-PLL re-administration by 15 °C at D47, D48 and D49 (MS19 to MS21) and 8.5 °C at D54, D55, D55 , D56, D61, D62, D63 (MS22 to MS27) for M47, M52, M53 and M54 mice. All these mice are still alive at D350. At D350, a treated mouse is euthanized and slices of its brain, located in the area of tumor cell implantation, are imaged by optical microscopy. The images of these slices reveal the absence of tumor or lesion, which suggests that these mice are totally cured. For mice of group 3, which receive BNF-Starch, tumor volumes continuously increase from 1.5 mm³ at D0 to 200 mm³ at D35. In addition, the mean survival time of these mice is estimated as 41±2 days (p = 0.338), a value similar to that of groups 1 and 2, suggesting that intratumor administration of BNF-Starch does not lead to significant antitumor activity. For mice belonging to group 4 receiving the BNF-starch and exposed to MS, the average tumor volume increases from D0 to D50. 7 mice (M28, M30, M31, M32, M33, M34 and M36) are prone to tumor growth delay compared with mice of groups 1, 2 and 3. In 2 mice (M29 and M35), total tumor disappearance without tumor regrowth is observed. Average temperatures of brain tumors increase by 8 °C at D5 (MS1), 0 °C at D6, D7, D12, D13, D14, D19, D20, D21, D26, D27, D28, D33, D34, D35, D40 , D41, D42 (MS2 to MS18), 5 °C at D47 (MS19) due to BNF-Starch re-administration and 0 °C at D48, D49, D54, D55, D56, D61, D62 and D63 (MS20 to MS27). 20% of these mice are still alive at D350 and average survival time is estimated at 125±43 days, significantly higher than in groups 1 and 2, but also lower than in group 6, indicating that anti-tumor activity is significant among these mice, but less pronounced than in group 5. We now turn to the histological analysis. First, for mice euthanized 6 hours after administration of M-PLL without MS, we observe that: i) PNN and M-PLL are located at the same site, designated as M-PNN, ii), M-PLL and PNN are not alone, iii), M-PNN occupy 100% of the tumor area, iv), there are no M-PNN outside of the tumor, v), there isn't any necrosis or apoptosis, vi) there are approximately 16000 M-PNN per mm² of tumor surface. Second, for mice euthanized 72 hours after M-PLL administration without MS, we observe that: i) there aren't any M-PNN or PNN alone, ii), M-PLL occupy 40 % of the tumor, iii), M-PLL seem to be internalized in tumor cells, iv), 14% of M-PLL are in the ventricles and 76% of IVI-PLL are in the tumor, v), there isn't any necrosis or apoptosis. Third, for mice that receive M-PLL and are euthanized 5h30 following a first MS, we observe that: i) there aren't any PNN or M-PLL alone, ii), PNN and M-PLL are located at the same site, designated as M-PNN, and are located at the tumor outskirt, iii), M-PNN occupy 13% of the tumor, iv), 70% of M-PNN are in the tumor and 30% of M-PNN are in the ventricles, v), necrotic and/or apoptotic cells are observed within 17% of the tumor area, vi), M-PLL appear to be internalized in tumor cells, vii), there are 28000 M-PNN per mm² of tumor surface. Fourth, for mice euthanized 24 hours after a third MS, we observe that: i), there aren't any PNN or M-PNN in the brain, ii), M-PLL occupy 17% of the tumor, iii), M-PLL are located at tumor outskirt, iv), M-PLL appear internalized in tumor cells. Fifth, for mice euthanized 6 hours after BNF-Starch administration without MS, we observe that: i), there is no PNN, ii), BNF-Starch occupy 12% of tumor surface and are located at tumor center, iii), there aren't any BNF-Starch outside of the tumor, iv), BNF-Starch appear internalized in tumor cells, v), necrotic and/or apoptotic cells occupy 23% of the tumor. Sixth, for mice euthanized 72 hours following BNF-Starch administration, we observe that: i), there aren't any PNN, ii), BNF-Starch occupy 53% of the tumor surface, iii), BNF-Starch are located at the tumor outskirt, iv), BNF-Starch seem internalized in tumor cells, v), BNF-Starch are not located outside of the tumor, vi), necrotic and/or apoptotic cells occupy 4% of the tumor.

Seventh, for mice euthanized 5h30 minutes after the first MS, we observe that: i), there isn't any PNN, ii), BNF-Starch occupy 10% of the tumor area, iii), BNF-Starch are located next to the tumor, iv), there aren't any BNF-Starch in the ventricles, v), BNF-Starch seem internalized in tumor cells. Eighth, for mice euthanized 24 hours following the third SM, we observe that: i) there isn't any PNN, ii), BNF-Starch occupy 27% of the tumor area, iii), BNF-Starch are near the tumor, iv) there aren't any BNF-starch in the ventricles, v), BNF-Starch seem internalized in tumor cells. We can conclude from this example that: i) it is possible to completely destroy U87-Luc tumors implanted in the brains of mice with one or two administration(s) of a suspension containing 500 µg in iron of M-PLL followed by MS, ii), M-PLL and PNN are located at the same site, *i.e*. separated by less than 1 µm, suggesting that M-PLL attract PNN, iii), antitumor activity is observed by applying the AMF in the absence of heat production provided that the tumor was previously heated, iv), the administration of M-PLL results in the appearance of M-PNN 6 hours following M-PLL administration where the number of M-PNN is higher in the presence (28161 M-PNN per mm² of tumor) than in the absence (15772 M-PNN per mm² of tumor) of MS, suggesting that the AMF increases the recruitment of PNN, iv), 72 hours after M-PLL administration in the absence of MS or in the presence of three MS, there are no PNN, suggesting that PNN disappear from the tumor (they are either destroyed or they migrate outside of the tumor) and the AMF can't reactivate them, probably due to the small amount of endotoxins at the M-PLL surface which is sufficient to cause a first neutrophil activation at 6 hours but not sufficient to cause a second neutrophil activation at 72 hours, v), 6 hours after M-PLL administration without MS, the amount of M-PNN is not much lower for M-PLL, at 15772 M-PNN per mm² of tumor, than for MC administration, at 18 413 M-PNN per mm² of tumor, whereas the amount of endotoxins is much larger in MC than in M-PLL suspensions, which may suggest that neutrophil activation is due to the gradual dissociation of endotoxins from M-PLL or MC at a dissociation rate, which is independent of the amount of endotoxin associated with the nanoparticles, vi), after administration of M-PLL, 12 hours after the first MS, 0 minute after the second MS, 24 hours after the third MS, it is possible to activate PNN and the amount of M-PNN is 27852 M-PNN per mm² of tumor while after administration of M-PLL, 24 hours after a third MS, it is not possible to activate the M-PNN, suggesting that when the amount of endotoxins at nanoparticle surface is higher, for MC, it is possible to activate neutrophils over a longer period of time by applying an AMF, possibly by dissociating endotoxins over a longer period of time in the presence of the AMF, vii), between 0, 4 and 12 hours following MC administration following the first MS, the amount of activated PNN increases from 4800 per mm² of tumor (0 hour) to 12800 per mm² of tumor (4 hours), to 27 852 per mm² of tumor (12 hours), suggesting that by applying the AMF, it is possible to obtain a progressive increase in PNN activation over time, which may be due to a gradual dissociation over time of endotoxins from MC, a behavior that is not observed when the AMF is applied between 6 and 72 hours following MC administration without MS, for which the number of M-PNN per mm² of tumor decreased from 18413 to 0, viii), 6 hours after M-PLL administration, the amount of M-PNN in the tumor decreases from 100% without MS to 70% in the presence of MS and 30% of M-PNN are in the ventricles following MS, which may suggest that the AMF produces M-PLL migration through the ventricles, possibly to remove M-PLL in the bloodstream, ix), anti-tumor activity leading to the total tumor disappearance is observed while M-PNN occupy 5h30 after a first MS or 24 hours following a third MS 70% and 17% of the tumor respectively, which suggests that partial occupancy of the tumor by M-PLL is sufficient to induce anti-tumor activity and therefore that an indirect mechanism occurs in which tumor cells directly destroyed by the treatment induce the destruction of the other tumor cells, ix), necrosis and/or apoptosis is observed in the presence of MS, suggesting that anti-tumor activity is due to the application of the AMF, x), M-PLL and BNF-Starch seem internalized in tumor cells, suggesting that internalization could play a role in antitumor activity, xi), in all conditions involving BNF-Starch administration, 6 or 72 hours following BNF-Starch administration without MS or 6 hours following BNF-Starch administration and a MS or 24 hours following BNF-Starch administration and a third MS, we don't observe PNN, suggesting that in the absence of endotoxins, PNN are not activated, xii), in the absence of PNN, BNF-Starch are not observed in the ventricles, which suggests that PNN may carry nanoparticles to the ventricles.

### Example 7: Dissociation of a substance from ferrimagnetic iron oxide nanoparticles (MCR400) exposed to radiation.

Three suspensions, containing either 400 µg/ml in iron oxide of MCR400, 125 µM of rhodamine B, or 400 µg/ml in iron oxide of MC mixed with 125 µM rhodamine B, are irradiated at doses between 0 and 1350 Gy using a Faxitron (160 kV, 6.3 mA without filter, 67.5 Gy/min). Luminescence intensity of suspensions containing rhodamine B alone or the supernate of the mixture of rhodamine B and MC do not vary or decrease when suspensions are irradiated. By contrast, when the MCR400 suspension is irradiated, the luminescence of its supernate, excited at 550 nm and detected at 576 nm, increases dramatically from 250 arbitrary units (a.u.) in the absence of irradiation to 600-950 a.u. in the presence of more than 250 Gy. These results suggest the dissociation of rhodamine B from MCR400 under irradiation. Moreover, emission wavelengths of the supernate of MCR400 and of rhodamine B free in solution remain unchanged at 576 nm, for the different radiation dose received by MCR400 or rhodamine B. This suggests that rhodamine B, free in solution or associated with magnetosomes, is not modified under irradiation. Increase in luminescence intensity of the supernate of the MCR400 suspension with irradiation is attributed to the dissociation of rhodamine B from MCR400 when MCR400 are irradiated. Similar behavior could potentially be observed with other substances than Rhodamine B such as immunogenic substances that could dissociate from magnetosomes under irradiation and cause a stimulation of the immune system.

### Example 8: Treatment of GL-261 tumors, grown subcutaneously under the skin of immunocompetent mice by administering suspensions containing BNF-Starch and montanide, MC, M-PEI and poly-IC, followed (or not) by the application of an AMF of frequency 198 KHz and average strength 9 mT.

Female "Black 6" mice are anesthetized with a ketamine/xelazine solution. 50 µl of a suspension containing 10⁵ GL-261 cells mixed in RPMI are administered subcutaneously on the right and left flanks of mice using a syringe. After induction of the subcutaneous tumors, animals are randomized in different groups. When tumor volumes reach 10 to 100 mm³, different suspensions are administered In an of the two tumors (right or left flank). The different suspensions of nanoparticles administered contain: i), 25 µl of BNF-Starch (Micromod: 10-00-102) at 20 mg/ml in iron mixed with 40 mg/mL of Montanide ISA51 (suspension 1), ii), 25µl of MC at 20 g/ml in iron with an endotoxin concentration of 18000 to 150000 EU/mg/mL (suspension 2), iii), 25 µl of M-PEI at 20 mg/ml in iron with an endotoxin concentration below 50 EU/mg/mg mixed with 10 mg/mL of polyI:C (suspension 3).

Groups 1 and 2 contain nine mice each and receive suspension 1 without MS (group 1) or with 15 MS (group 2). Groups 3 and 4 contain 7 mice each and receive suspension 2 without MS (group 3) or with 15 MS (group 4). Groups 5 and 6 contain 6 mice each and receive suspension 3 without MS (group 5) or with 15 MS (group 6). Each MS consists in the application of an AMF of average strength 9 mT and frequency of 198 kHz during 30 minutes. MS are applied three times a week during 5 weeks. We observe that: i), in groups 1 and 2, all tumors located at the non-administered flank reach 100 mm³ at D25 and tumor volume decreases to 0 mm³ are not observed, two tumors located in the administered flank increase to 100 mm³ between D5 and D10 and then decrease to 0 mm³ at D20 (group 1), one tumor located in the administered flank increases to 300 mm³ at D6, decreases to 0 mm³ at D27-D34 and then increases again to 250 mm3 at D43 (group 2) while other tumors of the administered flanks don't decrease to 0 mm³ in groups 1 and 2, suggesting partial antitumor activity in groups 1 and 2 in the administered flanks, where this activity is not enhanced by the application of the AMF, ii), in groups 3 and 4, a very similar behavior is observed between the administered and non-administered flanks in each mouse, *i.e*. in 2 mice of group 3 and 2 mice of group 4, both administered and non-administered tumor increase to 5-50 mm³ at D5-D10, then decrease to 0 mm³ at D15-D22, then remain at 0 mm³ between D22 and D75 and tumor disappearance is observed on both flanks, in 1 mouse of group 3, both administered and non-administered tumors do not reach 100 mm³ at D25 and tumor growth delay is observed on both flanks, In an mouse of group 4, the administered tumor does not reach 100 mm³ at D25 while the non-administered tumor does not grow, suggesting that the antitumor activity of the administered tumor can prevent the growth of the non-administered tumor, in 4 mice of group 3 and in 3 mice of group 4, nor tumor growth delay nor tumor disappearance is observed on both flanks, iii), in groups 5 and 6, a very similar behavior is observed between the left and right flanks, in 3 mice of group 5 and 1 mouse of group 6, administered tumors grow to 20-100 mm³ at D7-D22 and then decrease to 0 mm³ at D22-D40 while non administered tumors do not grow. Moreover, two groups of 10 mice receiving in GL-261 tumors of mean volumes 80 mm³ a suspension containing 25 µg in iron of M-PLL per mm³ of tumor or 25 µg in iron of BNF-Starch per mm³ of tumor are not prone to tumor growth delay or tumor disappearance during the month following the administration of the suspensions, suggesting that in groups 1 to 4, the presence of the immunogenic substance in suspensions 1 and 2 induces anti-tumor activity. During the various treatments and in particular during MS, we measure with a thermocouple and an infrared camera that the tumor temperature does not increase. We can conclude from this example that: i) tumor disappearance is observed when tumors receive either of suspension 1 to 3, suggesting that suspensions 1 to 3 lead to anti-tumor activity, ii), tumor disappearance or tumor growth delay is not more pronounced in the presence than in the absence of AMF application, suggesting that AMF application does not increase the anti-tumor activity, maybe due to a too weak applied AMF and/or to a too small quantity of administered nanoparticles, preventing a measurable tumor temperature increase, iii), treatments with MC or M-PEI and poly:IC lead to similar behaviors on both administered and non-administered flanks, suggesting the activation of an indirect mechanism of tumor destruction, such as an immune mechanism, in which anti-tumor activity at the site of MC or M-PEI and poly:IC induces anti-tumor activity in another site that can be remote from the first site, iv), the presence of an immunogenic substance seems needed to induce antitumor activity since similarly treated tumors with suspensions of BNF-Starch or non-immunogenic M-PLL do not display any anti-tumor activity.

### Example 9: Treatment of GL-261 tumors, grown subcutaneously under the skin of immunocompetent mice by administration of different suspensions containing either M-PLL or M- PLL mixed with MPLA followed (or not) by the application an AMF.

20 µl of a suspension containing 2.10⁶ GL261 cells are administered subcutaneously on the left flank of female "Black 6" mice at D0. After 15 days, when tumors reach 50 mm³ on average, mice are anaesthetized with Ketamine/Xylazine and 25 µl of different suspensions containing M-PLL at 20 mg/ml in iron (suspension 1), M-PLL at 20 mg/ml in iron mixed with 40 µg of MPLA (suspension 2) or 5% glucose (solution 3) are administered at the tumor center. Groups 1 and 2, containing 6 and 7 mice respectively, receive solution 3 at D0. Groups 3 and 4 contain 7 mice each and receive suspension 1 at D0. In groups 5 and 6, containing 6 and 8 mice each, mice receive suspension 2 at D0. Group 1, 3 and 5 are not treated after nanoparticle or glucose administration while group 2, 4 and 6 are exposed to 15 MS following administration. During MS, an AMF of average strength 30 mT and frequency 200 kHz is applied during 30 minutes and MS are carried out three times a week on anesthetized mice. During MS, tumor temperature is measured using a thermocouple and an infrared camera. It does not increase for groups 1, 2, 3 and 5 while it increases to an average of 43 °C in groups 4 and 6. We observe that: i) in group 1, all tumors reach 1000 mm³ at D25 and there is no tumor growth delay or tumor disappearance, ii), in group 2, 2 mice are prone to tumor growth delay with tumor volume below 500 mm³ at D25, iii) in group 3, 1 mouse is prone to tumor growth delay with a tumor volume below 500 mm³ at D25 and 1 tumor grows to 50 mm³ between D0 and D10 and then disappears at D20, iv), in group 4, 3 tumors are prone to tumor growth delay and have a volume of less than 500 mm³ at D25 and 1 tumor reaches 50 mm³ between D0 and D20 then disappears at D20, iv) in group 5, tumors of all mice reach 1000 mm³ at D25 and no tumor growth delay is observed, v) in group 6, 2 tumors are prone to tumor growth delay and have a volume below 500 mm³ at D25 and 1 tumor reaches 50 mm³ between D0 and D20 and then disappears at D20.

We can conclude from this example that: i), antitumor activity is observed when tumors are exposed to the AMF without M-PLL administration, when M-PLL are administered without AMF application or when M-PLL are administered and the AMF is applied, the last situation leading to strngest anti-tumor activity, ii), treatment with M-PLL and MLA does not seem more efficient than that with only M-PLL, suggesting that combining an immunogenic substance and a nanoparticle does not necessarily increase the efficacy of destruction of the primary tumor, iii), in the presence of the AMF leading to tumor temperature increase, treatment appears more efficient than in the absence of AMF.

### Example 10: Treatment of GL-261 tumors, grown subcutaneously under the skin of immunocompetent mice by administration of suspensions containing M-PLL or BNF-Starch followed (or not) by the application an AMF.

100 µl of suspensions containing ∼10⁷ cells are administered subcutaneously in the left flank of 6 to 8 weeks old C57/BL6 mice, anesthetized with isoflurane. 10 days after tumor cell implantation, at D0, when tumours reach 50 to 150 mm³, 6 groups, containing 10 mice each, are treated as follows: Group 1 receives an intratumor administration of 25 to 75 µl of a suspension containing 25 µg in iron of M-PLL per mm³ of tumor, where the M-PLL suspension is concentrated at 50 mg in iron per mL. M-PLL administration is followed by a series of 11 to 15 MS at D0, D2, D4, D7, D9, D 11, D14, D16, D18, D21, D23, D25, D28, D30, D32. MS, which last 30 minutes, consist either in a first magnetic treatment during which intratumor temperature is maintained at 43-46 °C by applying an AMF of 198 kHz and strength varied between 11 and 27 mT or in a second magnetic treatment during which intratumor temperature can not reach 43-46 °C and an AMF of 198 kHz and strength of 27 mT is applied. Group 2 receives an intratumor administration of 32 to 65 µl of a suspension containing 25 µg in iron of M-PLL per mm³ of tumor, where the M-PLL suspension is concentrated at 50 mg in iron per mL. Group 3 receives an intratumor administration of 32 to 47 µl of a suspension containing 25 µg in iron of BNF-Starch per mm³ of tumor, where the BNF-Starch suspension is concentrated at 50 mg in iron per mL. BNF-Starch administration is followed by a series of 7 to 15 MS at D0, D2, D4, D7, D9, D11, D14, D16, D18, D21, D23, D25, D28, D30, D32. MS, which last 30 minutes, consist either in a first magnetic treatment during which intratumor temperature is maintained at 43-46 °C by applying an AMF of 198 kHz and strength varied between 22 and 31 mT or in a second magnetic treatment during which intratumor temperature can not reach 43-46 °C and an AMF of 198 kHz and strength of 27 mT is applied. Group 4 receives an intratumor administration of 20 to 50 µl of a suspension containing 25 µg in iron of BNF-Starch per mm³ of tumor, where the BNF-Starch suspension is concentrated at 50 mg in iron per mL. Group 5 receives an intratumor administration of 50µl of a 5% glucose solution followed by 11 MS at D0, D2, D4, D7, D9, D11, D14, D16, D18, D21, D23. During each MS, a magnetic field frequency 198 kHz and average strength 27 mT was applied during 30 minutes. Group 6 receives an intratumor administration of 50 µl of a 5 % glucose solution.

In group 1, the efficacy of a M-PLL suspension to eradicate subcutaneous GL-261 tumors using magnetic hyperthermia treatment is evaluated by administering M-PLL at the center of GL-261 glioma tumors and by exposing mice to a first MS during which an AMF of 198 kHz and strength 11 to 27 mT is applied during 30 minutes. For a mouse with a typical behavior, tumor temperature rapidly increases from 29 °C before AMF application to 46 °C after 2 minutes of application of an AMF of 19 mT and then saturates at 45 °C during the remaining 28 minutes of application of the same AMF. The SAR of 40 W/gFe, deduced from the initial slope of the temperature variation with time, ΔT/δt = 0,24 °C/Sec, is about 25 % lower than the SAR measured in the gel. This difference could be due to the lower strength of the applied magnetic field or possibly to M-PLL aggregation within a part of the tumor following AMF application. Spatial temperature distributions, measured at the tumor surface with an infra-red camera shows that within 2 to 3 mm surrounding the tumor center, the temperature remains between 43 and 46°C. This behavior suggests that mild hyperthermia occurs within a mean volume of about 100 mm³, close to the mean tumor volume of 80 mm³. First MS is followed by 10 to 14 additional MS. The number of mice in which intratumor temperature can reach 43-46 °C by applying an AMF of 198 kHz and strength 11 to 27 mT decreases from 10 during the first MS at D0 down to 4 during the tenth MS at D21, remaining above 40% of the total number of mice.

Due to the loss of heating efficacy and to tumor growth of more than 25 % from two successive tumor size measurements, a second M-PLL administration at a quantity of 25 µg of M-PLL in iron per mm³ of tumor is carried out in 4 mice. To examine anti-tumor efficacy on mice belonging to group 1, variations of the tumor volume were measured during the days following M-PLL administration and animal survival rates were estimated. Compared with mice belonging to group 2 receiving only M-PLL without AMF or those of groups 5 and 6 receiving glucose in the absence or in the presence of an AMF respectively, we observe that 5 mice belonging to group 1 are prone to tumor growth delay. In the remaining 5 mice, the tumor disappears completely at D2, D25, D7, D7 and D11. In these mice, trace of the tumor are not visible 250 days following M-PLL administration, suggesting that they are fully cured. For mice belonging to group 1, where M-PLL suspension is re-administered at D11, D14, D 16 and D18, a decrease in tumor volume is observed during the days following re-administration without however yielding complete remission. These mice are euthanized at D25, D28, D30 and D44. For mice belonging to groups 2 and 5, which receive the M-PLL suspension without magnetic treatment or are exposed to the second magnetic treatment without M-PLL administration, tumor volumes increase continuously without any sign of anti-tumor activity, suggesting that the latter is due to M-PLL administration followed by MS and not solely to the M-PLL administration. Further support for anti-tumor activity is provided by survival rate estimates, which are 140 ± 37 days for mice belonging to group 1, much longer than 12 ± 2 days found for mice belonging to groups 2, 5 and 6. Optical images of tumor sections stained with Perls prussian blue for a mice receiving an intra-tumor administration of M-PLL without and with 1 MS and euthanized 6 hours following M-PLL administration reveal that: i), the percentage of M-PLL within the tumor (the surface occupied by M-PLL divided by the tumor surface) decreases from 41% without MS down to 21% with MS and, ii), the percentage of necrotic area (the surface occupied by necrotic areas divided by the tumor surface) within the turrior increases from 21% without MS up to 30% with MS. Similarly, for mice receiving M-PLL without or with MS and euthanized 72 hours following M-PLL administration, optical images reveal that: i), the percentage of M-PLL within the tumor decreases from 34% without MS down to 29% with MS and, ii), the percentage of necrotic areas within the tumor increases from 15% without MS up to 26% with MS. This suggests that AMF application reduces percentage of occupation of M-PLL in the tumor and increases the percentage of necrotic areas within the tumor. Outside of dense dark areas where M-PLL are located, iron possibly coming from M-PLL is not detected, suggesting that M-PLL are concentrated in part of the tumor.

Necrosis may not be the only mechanism responsible for tumor cell death when mice are treated by M-PLL administration followed by multiple since the percentage of necrotic areas in the tumor is not higher for mice treated with M-PLL than for those receiving glucose. In the site outside of the tumor where M-PLL are administered and exposed to two MS, very few necrotic areas are observed, essentially located in the surrounding skin. No necrotic area is observed in fatty or muscle tissues surrounding the tumor, suggesting that the treatment does not induce any significant damage to healthy tissues surrounding the tumor. This behavior may be explained by the temperature distributions, which shows that temperatures above 43°C, possibly inducing tissue damages, are not measured outside of the tumor and by tumor cells being more sensitive to heat than normal cells at these temperatures.

In group 3, a suspension of BNF-Starch was also administered at the center of GL-261 glioma tumors and mice were then exposed to a first MS during which an AMF of 198 kHz and strength 22 to 31 mT was applied during 30 minutes. For a typical mouse receiving this treatment and belonging to group 3 (M29), tumor temperature, measured at tumor center, increased up to 44 °C following the application of an AMF of a higher strength of 22 to 31 mT than for M-PLL.

The SAR deduced from the initial slope of the temperature variation with time, ΔT/δt = 0.155 °C/sec., was also lower than that estimated for M-PLL, indicating less efficient heating with BNF-Starch than with M-PLL. Compared with M-PLL, the temperature distribution at tumor surface, measured 10 minutes after the beginning of the session, displays a lower average temperature spread across the tumor. Indeed, mild hyperthermia with temperatures above 43°C was reached within 2 mm (< 50 mm³) surrounding the tumor center, i.e. within a lower percentage of tumor volume of 50 % than with M-PLL. Between MS 2 and MS 10, the number of mice in which the intra-tumor temperature could reach 43-46°C decreased more rapidly with the BNF-Starch, from 3 at MS 2 to 1 at MS 10, than with M-PLL where it decreased from 9 at MS 2 to 4 at MS 10. This behavior is observed despite the fact that the number of mice that received a second nanoparticle administration is larger for the BNF-Starch (6 mice) than for the M-PLL (4 mice). It can be concluded that increasing the magnetic field strength and the quantity of nanoparticles administered while using nanoparticles of lower SAR such as BNF-Starch does not necessarily enable to reach the same heating efficacy than that observed with nanoparticles of higher SAR such as M-PLL. Compared with mice receiving BNF-Starch (group 3) or glucose in the absence (group 6) or presence (group 5) of an AMF, tumor growth of 6 mice belonging to group 3 is delayed. Moreover, the tumor disappears fully in 2 mice at D14 and in 1 mouse at D23. The mean survival rate of mice belonging to this group is estimated at 69 ± 30 days, a value which is larger than that of 10-14 days observed for the control groups but also much lower than that of 140 ± 37 days obtained with M-PLL. It suggests that anti-tumor activity is observed with the BNF-Starch nanoparticles, but that this activity is less pronounced than that observed with M-PLL, a behavior that we attribute to a lower heating efficacy and different biodistribution properties for BNF-Starch than for M-PLL. Optical images of tumor sections stained with Perls prussian blue for a mice receiving an intra-tumor administration of BNF-Starch without and with 1 MS and euthanized 6 hours following BNF-Starch administration reveal that: i), the percentage of BNF-Starch within the tumor (the surface occupied by BNF-Starch divided by the tumor surface) increases from 122% without MS up to 144% with MS and, ii), the percentage of necrotic area (the surface occupied by necrotic areas divided by the tumor surface) within the tumor increases from 8% without MS up to 17% with MS. Similarly, for mice receiving BNF-Starch without or with MS and euthanized 72 hours following BNF-Starch administration, optical images reveal that: i), the percentage of BNF-Starch within the tumor increases from 53% without MS up to 93% with MS and, ii), the percentage of necrotic areas within the tumor increases from 7% without MS up to 43% with MS. This suggests that AMF application increases the percentage of occupation of BNF-Starch in the tumor and increases the percentage of necrotic areas within the tumor. As a whole, BNF-Starch appear to distribute more homogeneously than M-PLL in the tumor and the application of the AMF increases the percentage of BNF-Starch distribution within the tumor, whereas the opposite behavior is observed with M-PLL.

### Example 11: Different cytotoxicity properties of M-PLL and BNF-Starch incrubated in the presence of healthy 3T3 and cancerous GL-261 cells during 24 hours.

MTT cytotoxicity assay of nanoparticles incubated with GL-261 cells follows that described in ACSNano, V. 5, P. 6279-6296 (2011) for MDA-MB-231 cells while that of nanoparticles incubated with 3T3 cells follows ISO 10993-5 standard. Assays are carried out in the absence of radiation.

Nanoparticle cytotoxicity on 3T3 cells (standard fibroblast cell line) is not observed for incubated suspensions containing M-PLL and BNF-Starch at concentrations in iron per mL of 87.5 µg/mL, 175 µg/mL, 250 µg/mL, 500 µg/mL and 1000 µg/mL. Indeed, in these cases, viability is always larger than 70% (ISO 10993-5:2009(E)). By contrast, when the same experiment is carried out on GL261 cells (tumor glioblastoma cells), cell viability is 70% for for the M-PLL suspension incubated at 250 µg/mL and is reduced to 0% for M-PLL incubated at 500 µg/mL and 1000 µg/mL. Concerning BNF-Starch, cell viability is below 70% for BNF-Starch suspension incubated at 1000 µg/mL. We conclude from this example that M-PLL and BNF-Starch are more cytotoxic towards tumor (GL-261) than healthy (3T3) cells and therefore that the presence of nanoparticles both in healthy and tumor tissues can potentially selectively destroy tumor tissues without destroying healthy tissues.

### Example 12: Comparison between the specific absorption rates of suspensions of chains of magnetosomes isolated from MSR-1 magnetotactic bacteria (MCh) mixed in water, uncoated magnetosome minerals (M) mixed in water, magnetosome minerals coated with citric acid (M-CA) mixed in water.

To prepare the suspension of MC, MSR-1 magnetotactic bacteria with an optical density of 50 at 565 nm were mixed with KOH at 2M and the mixture was heated during 30 minutes at 80°C under agitation at 150 rpm. MC were then selected magnetically by positioning a strong Neodinium magnet against the tube containing the mixture, by removing the supernate and by replacing it with PBS 10X. To prepare M, the suspension of MC was mixed with PBS and Triton and heated during one night at 50 °C, the suspension was placed against a Neodinium magnet, the supernate was removed and replaced by phenol, the suspension was heated at 60 °C during two hours, the supernate was removed and replaced by chloroform, the supernate was again removed and replaced by sterile water. To prepare the suspension containing M-CA, 200 mg of citric acid monohydrate (33114, Sigma) were dissolved in 6 mL of ultrapure water. pH of the solution was adjusted to 6 with NaOH.

Ultrapure water was added to the solution to obtain 10 mL of a solution at a concentration of 20 mg/mL and the solution was homogenized with a vortex. Under a sterile hood, the citric acid solution was filtered with a 0.45 µm filter. 630 µl of MC mixed in water at a concentration of 19 mg/mL were inserted in a 20 mL glass tube, the tube was positioned against a Neodinium magnet of 1.5 T, we waited until the supernate was transparent to remove the supernate. The supernate was replaced by 3.5 mL of an acid citric solution at 20 mg/mL at pH 6. Sterile apyrogen water was added to obtain 10 mL of a suspension of MC mixed with citric acid and water at a concentration in iron of 2 mg/mL. This suspension was placed in a sonicating bath (25 kHz) at 90 °C during 5 hours. The suspension was then washed twice-by positioning the tube containing the suspension against the same Neodinium magnet and by replacing water with sterile water. The suspension was then centrifugated at 12 000 rpm during 30 minutes and the supernate was removed and replaced by sterile water. Suspensions containing in 0.5 mL of water 5 mg of MC (suspension 1), 5 mg of M (suspension 2), or 5 mg of M-CA (suspension 3) are exposed during 1000 seconds to an alternating magnetic field of average strength 25 mT and frequency 198 kHz. From the slope of the initial temperature variation with time, ΔT/δt = 1.1 °C/sec. (suspension 1), ΔT/δt = 1.1 °C/sec. (suspension 2), 2.2 °C/sec. (suspension 3), we deduced SAR of 462 W/gFe (suspensions 1 and 2) and 924 W/gFe (suspension 3). For suspension 3, when the same experiment was carried out using 0.5 mg of M-CA mixed in 0.5 mL of water, we found a SAR of 226 W/gFe, indicating that the SAR decreases when it is measured at lower concentration. For suspension 3, when the same experiment was carried out using a suspension containing in 1.5 mL of water 15 mg of water, we found a SAR of 68 W/gFe, indicating that the SAR decreases when it is measured in a larger volume.

### Example 13: Estimate of the SAR of BNF-Starch in suspension as a function of BNF-Starch concentration and strength of the applied alternating magnetic field.

100 µl of suspensions containing various concentrations of BNF-Starch (1.4, 10, 14 or 20 mg in iron per mL) mixed in water are introduced in tubes of volume 250 µl and exposed to an alternating magnetic field of frequency 198 kHz and average strength of 25 mT or 30 mT. The thermocouple temperature probe is positioned at the bottom of the tube, we wait for 800 seconds that the temperature stabilizes before applying the alternating magnetic field during 15 minutes.

For the average magnetic field strength of 25 mT, the measured SAR are 37 W/gFe at 1.4 mg/mL, 40 W/gFe at 10 mg/mL, 45 W/gFe at 14 W/gFe, 57 W/gFe at 20 mg/mL. For the average magnetic field strength of 30 mT, the measured SAR are 75 W/gFe at 1.4 mg/mL, 125 W/gFe at 10 mg/mL, 105 W/gFe at 14 nig/mL, 112 W/gFe at 20 mg/mL. We can conclude from this experiment that the SAR increases when the nanoparticle concentration increases. For the average magnetic strength of 25 mT, the concentration of 20 mg/mL does not seem to be sufficient to reach the maximum SAR since the SAR increases continuously as a function of concentration. By contrast, for the average magnetic field strength of 30 mT, the maximum SAR seems to be reached for the concentration of 10 mg/mL and the saturating concentration is between 1.4 mg/mL and 10 mg/mL. We can also conclude that the SAR increases with increasing magnetic field strength since for any given tested concentration, the SAR is larger for an applied magnetic field of strength 30 mT than for that of strength 25 mT.

## Claims

1. A composition comprising at least one association of at least one magnetic narioparticle and at least one substance, wherein the association is activated by radiation, for use in a treatment by radiation for destroying at least one pathological cell in an individual, comprising administering the composition at a body site of an individual and performing at least one type of activation of the association by radiation to activate magnetic nanoparticles without producing any temperature increase, whereby the pathological cell destruction occurs at the administration site and away from this site beyond the association diffusion region.

2. The composition for use according to claim 1, wherein another type of activation by radiation is applied to produce a temperature increase.

3. The composition for use according to claim 1 or 2, wherein the association is immunogenic.

4. The composition for use according to any of claims 1 to 3, wherein the activation by radiation leads to the migration of the targeting cell towards the pathological site, wherein the targeting cell is a cell that specifically targets the pathological site.

5. The composition for use according to any of claims 1 to 4, wherein the association is in suspension or in a solid matrix and is dispersed homogenously before it is brought into contact with the targeting cell and wherein the association, when brought into contact with the targeting cell, redistributes under activation by radiation either by concentrating at a site comprising the targeting cell or by being removed from a site comprising the targeting cell, and wherein the redistributed association destroys at least one the pathological cell under activation by radiation.

6. A composition comprising at least one association of at least one nanoparticle and at least one substance for use in a method of diagnosis of the presence of at least one pathological cell in an individual, comprising:
- firstly, administering the.association at a site of the body of an individual wherein the presence of a least one the pathological cell is suspected,
- secondly, activating the association, thereby leading to the migration of the targeting cell towards the association,
- thirdly, detecting the targeting cell, in order to reveal the presence of at least one the pathological cell.

7. The composition for use according to any one of claims 1 to 6, wherein the targeting cell under activation by radiation migrates towards the administration or pathological site.

8. The composition for use according to any one of claims 1 to 7, wherein the targeting cell is a tumor cell or a cell infected by a pathogen.

9. The composition for use according to any one of claims 1 to 8, wherein the administered association is concentrated at a site comprising a portion of the total number of pathological cell comprised in an individual part at a concentration which is sufficient to enable the activation by the radiation.

10. The composition for use according to any one of claims 1 to 9, wherein the administered association is redistributed by the targeting cell, by being concentrated at the site comprising a portion of the total number of pathological cell comprised in an individual or by being removed from this site.

11. A composition comprising at least one association of two or more magnetic nanoparticles and a compound that stabilizes and/or coat the two or more magnetic nanoparticles and/or bind the two or more magnetic nanoparticles together, wherein the two or more magnetic nanoparticles without the compound possess a SAR of less than 200 W/gFe and wherein the association comprising the two or more nanoparticles and the compound possess a SAR larger than 200 W/gFe.

12. The composition according to claim 20, wherein the magnetic nanoparticle is synthetized by a living organism and the compound is not synthesized by this same living organism.

13. The composition according to claim 20, wherein the magnetic nanoparticle is synthesized chemically and has the same coercivity and/or remanent magnetization and/or saturating magnetization and/or a ratio between remanent magnetization and saturating magnetization and/or SAR than the coercivity and/or remanent magnetization and/or saturating magnetization and/or ratio between remanent magnetization and saturating magnetization and/or SAR of the magnetic nanoparticle synthesized by the living organism, and wherein the compound is not synthesized by the living organism.

14. The composition according to any one of claims 20 to 22, wherein the specific absorption rate of the association is larger than that of magnetic nanoparticle synthesized by the living organism and surrounded by a coating substance, which contains organic material also coming from the living organism.

15. The composition for use according to any one of claims 20 to 23, to attract, detect or destroy a cell.
